(19) Europäisches Patentamt
European Patent Office
Office européen des brevets

(11) EP 2 794 838 B1

(12) EUROPEAN PATENT SPECIFICATION

(45) Date of publication and mention of the grant of the patent:
**14.02.2018 Bulletin 2018/07**

(21) Application number: **12808368.0**

(22) Date of filing: **18.12.2012**

(51) Int Cl.:
***C11D 3/386*** (2006.01) ***C12N 9/54*** (2006.01)

(86) International application number:
**PCT/EP2012/075944**

(87) International publication number:
**WO 2013/092582 (27.06.2013 Gazette 2013/26)**

# (54) DETERGENT COMPOSITIONS COMPRISING SUBTILASE VARIANTS

REINIGUNGSMITTELZUSAMMENSETZUNGEN MIT SUBTILASEVARIANTEN

COMPOSITIONS DE DÉTERGENT COMPORTANT DES VARIANTS DE SUBTILASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2011 EP 11194520**

(43) Date of publication of application:
**29.10.2014 Bulletin 2014/44**

(60) Divisional application:
**17209243.9**

(73) Proprietor: **Henkel AG & Co. KGaA**
**40589 Düsseldorf (DE)**

(72) Inventors:
- **O'CONNELL, Timothy**
  **40547 Düsseldorf (DE)**
- **TONDERA, Susanne**
  **40597 Düsseldorf (DE)**
- **HELLMUTH, Hendrik**
  **40237 Düsseldorf (DE)**
- **WEBER, Thomas**
  **41541 Dormagen (DE)**
- **BESENMATTER, Werner**
  **2351 Wiener Neudorf (AT)**
- **BENIE, Astrid**
  **DK-3500 Vaerlose (DK)**
- **FRIIS, Esben**
  **DK-2730 Herlev (DK)**
- **OLLENDORFF MICHEELSEN, Pernille**
  **2500 Valby (DK)**

(56) References cited:
**WO-A1-00/37627** **WO-A1-2011/072117**
**WO-A2-2004/041979** **WO-A2-2009/149144**

- **DATABASE Geneseq [Online] 1 September 2011 (2011-09-01), "Subtilisin BPN' mutant P0521.", XP002680118, retrieved from EBI accession no. GSP:AZL29478 Database accession no. AZL29478**
- **NARINX E ET AL: "Subtilisin from psychrophilic antarctic bacteria: characterization and site-directed mutagenesis of residues possibly involved in the adaption to cold", PROTEIN ENGINEERING, OXFORD UNIVERSITY PRESS, SURREY, GB, vol. 10, no. 11, 1 November 1997 (1997-11-01), pages 1271-1279, XP002071383, ISSN: 0269-2139**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 794 838 B1

SEQUENCE LISTING

<110> Henkel AG & Co. KGaA

<120> Detergent compositions comprising subtilase variants

<130> PT019568

<150> EP11194520.0
<151> 2011-12-20

<160> 2

<170> PatentIn version 3.5

<210> 1
<211> 1149
<212> DNA
<213> Bacillus amyloliquefaciens

<400> 1

```
atgagaggca aaaaagtatg gatcagtttg ctgtttgctt tagcgttaat ctttacgatg        60
gcgttcggca gcacatcctc tgcccaggcg gcagggaaat caaacgggga aaagaaatat       120
attgtcgggt ttaaacagac aatgagcacg atgagcgccg ctaagaagaa agatgtcatt       180
tctgaaaaag gcgggaaagt gcaaaagcaa ttcaaatatg tagacgcagc ttcagctaca       240
ttaaacgaaa aagctgtaaa agaattgaaa aaagacccga gcgtcgctta cgttgaagaa       300
gatcacgtag cacatgcgta cgcgcagtcc gtgccttacg gcgtatcaca aattaaagcc       360
cctgctctgc actctcaagg ctacactgga tcaaatgtta aagtagcggt tatcgacagc       420
ggtatcgatt cttctcatcc tgatttaaag gtagcaggcg gagccagcat ggttccttct       480
gaaacaaatc ctttccaaga caacaactct cacggaactc acgttgccgg cacagttgcg       540
gctcttaata actcaatcgg tgtattaggc gttgcgccaa gcgcatcact ttacgctgta       600
aaagttctcg gtgctgacgg ttccggccaa tacagctgga tcattaacgg aatcgagtgg       660
gcgatcgcaa acaatatgga cgttattaac atgagcctcg gcggaccttc tggttctgct       720
gctttaaaag cggcagttga taaagccgtt gcatccggcg tcgtagtcgt tgcggcagcc       780
ggtaacgaag gcacttccgg cagctcaagc acagtgggct accctggtaa atacccttct       840
gtcattgcag taggcgctgt tgacagcagc aaccaaagag catctttctc aagcgtagga       900
cctgagcttg atgtcatggc acctggcgta tctatccaaa gcacgcttcc tggaaacaaa       960
tacggggcgt acaacggtac gtcaatggca tctccgcacg ttgccggagc ggctgctttg      1020
attctttcta agcacccgaa ctggacaaac actcaagtcc gcagcagttt agaaaacacc      1080
actacaaaac ttggtgattc tttctactat ggaaaagggc tgatcaacgt acaggcggca      1140
gctcagtaa                                                              1149
```

<210> 2
<211> 275
<212> PRT
<213> Bacillus amyloliquefaciens

<400> 2

```
Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160

Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
```

```
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275
```

## Description

### Reference to a Sequence Listing

This application contains a Sequence Listing in computer readable form, which is incorporated herein by reference.

### Background of the Invention

### Field of the Invention

The present invention relates to protease containing detergent compositions, especially detergent compositions comprising subtilase variants exhibiting alterations relative to the parent subtilase in one or more properties including: Wash performance, thermal stability, storage stability or catalytic activity. Further, the present invention relates to methods of producing said detergent compositions and to the use said detergent compositions in cleaning applications.

### Description of the Related Art

In the detergent industry enzymes have for more than 30 years been implemented in washing formulations. Enzymes used in such formulations comprise proteases, lipases, amylases, cellulases, mannosidases as well as other enzymes or mixtures thereof. Commercially the most important enzymes are proteases.
An increasing number of commercially used proteases are protein engineered variants of naturally occurring wild type proteases, Everlase®, Relase® Coronase®, Liquanase®, Ovozyme®, Polarzyme® and Kannase® (Novozymes a/s),Maxacal®, Properase®, Purafect®, FN2®, FN3® and FN4® (DuPont/Genencor International, Inc.).
Further, a number of variants are described in the art, such as in WO 2004/041979 (Novozymes A/S) which describes subtilase variants exhibiting alterations relative to the parent subtilase in e.g. wash performance, thermal stability, storage stability or catalytic activity. The variants are suitable for use in e.g. cleaning or detergent compositions.
A number of useful subtilase variants have been described many of which have provided improved activity, stability, and solubility in different detergents. In US6436690 (Brode III et.al) describes alteration in the loop 59 to 66 (BPN' numbering), in WO2009/149200 (Danisco US INC.) substitution at position 53 and 55 (BPN' numbering) is described. Further WO2002/31133 (Novozymes A/S) describes insertions in the loop 51-56 (BPN' numbering). However, various factors make further improvement of the proteases advantageous. The washing conditions keep changing e.g. with regards to temperature and pH and many stains are still difficult to completely remove under conventional washing conditions. Thus despite the intensive research in protease development there remains a need for detergent compositions with improved proteases.
Description of cited reference D1 (WO2011/072117) to be inserted in page 1, last paragraph, after the 3rd sentence of the description as filed:

> "WO2011/072117 discloses cold water proteases derived from BPN' subtilisin and their use in fabric and homecare products."

It is therefore an object of the present invention to provide detergent compositions with variants of a protease with improved properties compared to its parent protease.

### Summary of the Invention

The present invention relates to a method for producing a detergent composition which comprises the step of adding a protease variant which was obtained by a method comprising introducing into a parent subtilase a deletion at one positions corresponding to positions 53 of the mature polypeptide consisting of SEQ ID NO: 2 and an additional deletion of one or more amino acids selected from the group consisting of Ser, Glu, Thr, Asn or Pro in the loop corresponding to positions 54, 55, 56 or 57 of the mature polypeptide consisting of SEQ ID NO: 2, wherein the variant has at least 95% identity to SEQ ID NO: 2. The present invention also relates to a detergent composition comprising a protease variant comprising a deletion at one amino acid corresponding to position 53 of the mature polypeptide consisting of SEQ ID NO: 2 and further comprising one or more deletions at positions corresponding to positions 54, 55, 56 or 57 of the mature polypeptide consisting of SEQ ID NO: 2, wherein the variant has protease activity and the variant has at least 95% and less than 100% sequence identity to SEQ ID NO:2. The invention further relates to the use of detergent compositions according to the invention in cleaning processes such as laundry and/or dish wash.

## Definitions

**Protease:** The term "protease" is defined herein as an enzyme that hydrolyses peptide bonds. It includes any enzyme belonging to the EC 3.4 enzyme group (including each of the thirteen subclasses thereof). The EC number refers to Enzyme Nomenclature 1992 from NC-IUBMB, Academic Press, San Diego, California, including supplements 1-5 published in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively.

**Protease activity:** The term "protease activity" means a proteolytic activity (EC 3.4). Proteases of the invention are endopeptidases (EC 3.4.21). There are several protease activity types: The three main activity types are: trypsin-like where there is cleavage of amide substrates following Arg or Lys at P1, chymotrypsin-like where cleavage occurs following one of the hydrophobic amino acids at P1, and elastase-like with cleavage following an Ala at P1. For purposes of the present invention, protease activity is determined according to the procedure described in "Materials and Methods" below. The subtilase variants of the present invention have at least 20%, e.g., at least 40%, at least 50%, at least 60%, at least 70%, at least 80%, at least 90%, at least 95%, and at least 100% of the protease activity of the mature polypeptide of SEQ ID NO:2.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Improved property:** The term "improved property" means a characteristic associated with a variant that is improved compared to the parent or compared to a protease with SEQ ID NO: 2, or compared to a protease having the identical amino acid sequence of said variant but not having the alterations at one or more of said specified positions. Such improved properties include, but are not limited to, wash performance, protease activity, thermal activity profile, thermostability, pH activity profile, pH stability, substrate/cofactor specificity, improved surface properties, substrate specificity, product specificity, increased stability, improved stability under storage conditions, and chemical stability.

**Improved wash performance:** The term "improved wash performance" is defined herein as a protease variant or a detergent composition comprising said protease variant displaying an alteration of the wash performance relative to the wash performance of the parent subtilase or the corresponding detergent composition, relative to a protease with SEQ ID NO: 2 or the corresponding detergent composition, or relative to a protease having the identical amino acid sequence of said variant but not having the alterations at one or more of said specified positions or the corresponding detergent composition, e.g. by increased stain removal which is particularly preferred. The term "wash performance" includes wash performance in laundry but also e.g. in dish wash. The wash performance may be quantified as described under the definition of "wash performance" herein.

**Improved protease activity:** The term "improved protease activity" is defined herein as an altered protease activity (as defined above) of a protease variant displaying an alteration of the activity relative (or compared) to the activity of the parent subtilase, or compared to a protease with SEQ ID NO: 2, or relative to a protease having the identical amino acid sequence of said variant but not having the alterations at one or more of said specified positions, by increased protein conversion.

**Improved thermal activity:** The term "improved thermal activity" means a variant displaying an altered temperature-dependent activity profile at a specific temperature relative to the temperature-dependent activity profile of the parent or relative to a protease with SEQ ID NO: 2. The thermal activity value provides a measure of the variant's efficiency in enhancing catalysis of a hydrolysis reaction over a range of temperatures. A variant is stable and retains its activity in a specific temperature range, but becomes less stable and thus less active with increasing temperature. Furthermore, the initial rate of a reaction catalyzed by a variant can be accelerated by an increase in temperature that is measured by determining thermal activity of the variant. A more thermoactive variant will lead to an increase in enhancing the rate of hydrolysis of a substrate by an enzyme composition thereby decreasing the time required and/or decreasing the enzyme concentration required for activity. Alternatively, a variant with a reduced thermal activity will enhance an enzymatic reaction at a temperature lower than the temperature optimum of the parent defined by the temperature-dependent activity profile of the parent.

**Low stringency conditions:** The term "low stringency conditions" means for probes of at least 100 nucleotides in length, prehybridization and hybridization at 42°C in 5X SSPE, 0,3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. In the invention, the mature polypeptide corresponds to the amino acid sequence with SEQ ID NO: 2.

**Mature polypeptide coding sequence:** The term "mature polypeptide coding sequence" means a polynucleotide that encodes a mature polypeptide having protease activity. In the invention, the mature polypeptide coding sequence is nucleotides 322 to 1146 of SEQ ID NO: 1 based on the SignalP (Nielsen et al., 1997, Protein Engineering 10: 1-6)] that predicts nucleotides 1 to 90 of SEQ ID NO: 1 encodes a signal peptide.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and either 35% formamide, following standard Southern blotting procedures for 12 to 24 hours. The carrier material is finally washed three times each for 15 minutes using 2X SSC, 0.2% SDS at 60°C.

**Mutant:** The term "mutant" means a polynucleotide encoding a variant.

**Parent:** The term "parent" means a protease to which an alteration is made to produce the enzyme variants of the present invention. Thus the parent is a protease having the identical amino acid sequence of said variant but not having the alterations at one or more of said specified positions. It will be understood, that in the present context the expression "having identical amino acid sequence" relates to 100% sequence identity. The parent may be a naturally occurring (wild-type) polypeptide or a variant thereof. In a particular embodiment the parent is a protease with at least 95%, at least 96%, at least 97%, at least 98%, at least 99%, or 100% identity to a polypeptide with SEQ ID NO: 2.

**Sequence Identity:** The relatedness between two amino acid sequences or between two nucleotide sequences is described by the parameter "sequence identity". For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/ (Length of Alignment – Total Number of Gaps in

**Substantially pure variant:** The term "substantially pure variant" means a preparation that contains at most 10%, at most 8%, at most 6%, at most 5%, at most 4%, at most 3%, at most 2%, at most 1%, and at most 0.5% by weight of other polypeptide material with which it is natively or recombinantly associated. Preferably, the variant is at least 92% pure, e.g., at least 94% pure, at least 95% pure, at least 96% pure, at least 97% pure, at least 98% pure, at least 99%, at least 99.5% pure, and 100% pure by weight of the total polypeptide material present in the preparation. The variants of the present invention are preferably in a substantially pure form. This can be accomplished, for example, by preparing the variant by well known recombinant methods or by classical purification methods.

**Variant:** The term "variant" means a polypeptide having protease activity comprising an alteration, *i.e.,* a substitution, insertion, and/or deletion, at one or more (or one or several) positions compared to its parent which is a protease having the identical amino acid sequence of said variant but not having the alterations at one or more of said specified positions. A substitution means a replacement of an amino acid occupying a position with a different amino acid; a deletion means removal of an amino acid occupying a position; and an insertion means adding amino acids e.g. 1 to 10 amino acids , preferably 1-3 amino acids adjacent to an amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100

nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Wash performance:** The term "wash performance" is used as an enzyme's or detergent's ability to remove stains present on the object to be cleaned during e.g. wash, such as laundry or hard surface cleaning. The wash performance may be quantified by calculating the so-called intensity value (Int) defined in AMSA assay as described in Materials and methods herein. See also the wash performance test in Example 2 herein. Further, the wash performance, especially the wash performance of a detergent composition according to the invention, may be determined by the reference washing test described below. Especially the wash performance of a liquid detergent composition according to the invention is determined by said reference washing test. See also Example 3 herein.

**Wild-Type protease:** The term "wild-type protease" means a protease expressed by a naturally occurring organism, such as a bacterium, archaea, yeast, fungus, plant or animal found in nature. An example of a wild-type protease is BPN' i.e. SEQ ID NO: 2.

## Conventions for Designation of Variants

For purposes of the present invention, the mature polypeptide disclosed in SEQ ID NO: 2 is used to determine the corresponding amino acid residue in another subtilisin. The amino acid sequence of another subtilisins is aligned with the mature polypeptide disclosed in SEQ ID NO: 2, and based on the alignment, the amino acid position number corresponding to any amino acid residue in the mature polypeptide disclosed in SEQ ID NO: 2 is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

Identification of the corresponding amino acid residue in another subtilisin can be determined by an alignment of multiple polypeptide sequences using several computer programs including, but not limited to, MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537:_39-64; Katoh and Toh, 2010, Bioinformatics 26:_1899-1900), and EMBOSS EMMA employing ClustalW (1.83 or later; Thompson et al., 1994, Nucleic Acids Research 22: 4673-4680), using their respective default parameters.

When the other enzyme has diverged from the mature polypeptide of SEQ ID NO: 2 such that traditional sequence-based comparison fails to detect their relationship (Lindahl and Elofsson, 2000, J. Mol. Biol. 295: 613-615), other pairwise sequence comparison algorithms can be used. Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide, and such models can be assessed for accuracy using a variety of tools developed for that purpose.

For proteins of known structure, several tools and resources are available for retrieving and generating structural alignments. For example the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (Shindyalov and Bourne, 1998, Protein Engineering 11: 739-747), and implementation of these algorithms can additionally be utilized to query structure databases with a structure of interest in order to discover possible structural homologs (*e.g.,* Holm and Park, 2000, Bioinformatics 16: 566-567).

In describing the variants of the present invention, the nomenclature described below is adapted for ease of reference. The accepted IUPAC single letter or three letter amino acid abbreviation is employed.

Substitutions. For an amino acid substitution, the following nomenclature is used: Original amino acid, position, substituted amino acid. Accordingly, the substitution of threonine at position 226 with alanine is designated as "Thr226Ala" or "T226A". Multiple mutations are separated by addition marks ("+"), commas or by space, *e.g.,* "Gly205Arg + Ser411

Phe" or "G205R + S411F", "G205R, S411F", "G205R S411F" representing substitutions at positions 205 and 411 of glycine (G) with arginine (R) and serine (S) with phenylalanine (F), respectively.

Deletions. For an amino acid deletion, the following nomenclature is used: Original amino acid, position, *. Accordingly, the deletion of glycine at position 195 is designated as "Gly195*" or "G195*". Multiple deletions are separated by addition marks ("+"), *e.g.,* "Gly195* + Ser411*" or "G195* + S411*".

Insertions: The insertion of an additional amino acid residue such as e.g. a lysine after G195 may be indicated by: Gly195GlyLys or G195GK. Alternatively insertion of an additional amino acid residue such as lysine after G195 may be indicated by: *195aL. When more than one amino acid residue is inserted, such as *e.g.* a Lys, and Ala after G195 this may be indicated as: Gly195GlyLysAla or G195GKA. In such cases, the inserted amino acid residue(s) may also be numbered by the addition of lower case letters to the position number of the amino acid residue preceding the inserted amino acid residue(s), in this example: *195aK *195bA. In the above example, the sequences 194 to 196 would thus be:

| | | | | | |
|---|---|---|---|---|---|
| | 194 | 195 | 196 | | |
| Savinase | A - | G - | L | | |
| | 194 | 195 | 195a | 195b | 196 |
| Variant | A - | G - | K - | A - | L |

In cases where a substitution and an insertion occur at the same position this may be indicated as S99SD+S99A or in short S99AD. The same modification may also be indicated as S99A + *99aD.

In cases where an amino acid residue identical to the existing amino acid residue is inserted it is clear that degeneracy in the nomenclature arises. If for example a glycine is inserted after the glycine in the above example this would be indicated by G195GG or *195aGbG. The same actual change could just as well be indicated as A194AG or *194aG for the change from

| | | | | |
|---|---|---|---|---|
| | 194 | 195 | 196 | |
| Savinase | A - | G - | L | |
| | 194 | 195 | 195a | 196 |
| Variant | A - | G - | G - | L |
| | 194 | 194a | 195 | 196 |

Such instances will be apparent to the skilled person and the indication G195GG and corresponding indications for this type of insertions are thus meant to comprise such equivalent degenerate indications.

Different alterations. Where different alterations can be introduced at a position, the different alterations are separated by a comma, *e.g.,* "Arg170Tyr,Glu" represents a substitution of arginine at position 170 with tyrosine or glutamic acid. Thus, "Tyr167Gly,Ala + Arg170Gly,Ala" designates the following "Tyr167Gly+Arg170Gly", "Tyr167Gly+Arg170Ala", "Tyr167Ala+Arg170Gly", and "Tyr167Ala+Arg170Ala".

**Detailed Description of the Invention**

Previously unanticipated, the inventors have found that detergent compositions comprising protease variants, especially subtilase variants, containing one or more deletion and/or substitution in the positions 53-57 have improved wash performance compared to an otherwise identical detergent composition with a protease having the identical amino acid sequence of said variant but not having the alterations at one or more of said specified positions or compared to a protease with SEQ ID NO: 2. The amino acids corresponding to positions 53-57 of SEQ ID NO: 2 form part of a loop, which connects a β-sheet with the α-helix that contains H64, which is part of the catalytic triade D32, H64 and S221 of the active site. The amino acid sequence of the α-helix is very much conserved among wild-type proteases of the S8-type. Also the β-sheet is conserved. However the connecting loop has a high sequence variety. This is exemplified with the following alignment of the two S8 proteases BPN' (SEQ ID NO: 2) and Savinase (a protease well known in the art) of amino acid sequence positions 51-70:

```
51   56     63     70
VPSETNPFQDNNSHGTHVAG  BPN'
|| |    || | |||||||
VPGEP STQDGNGHGTHVAG  Savinase
```

New protease variants containing a deletion in the positions 53-57 (BPN' numbering), as well as variants containing the deletion together with one or several substitutions in the loop region were generated and tested for wash performance as described in "Material and Methods" and the inventors demonstrate that one or more deletions of one or more amino acid at a position corresponding to positions 53, 54, 55, 56 or 57 of the mature polypeptide of SEQ ID NO: 2 significantly improved wash performance compared to a protease having the identical amino acid sequence of said variant but not having the alterations at one or more of said specified positions or compared to a protease with SEQ ID NO: 2. Thus the invention relates to a method for producing a detergent composition which comprises the step of adding a protease variant which was obtained by a method comprising introducing into a parent subtilase a deletion at one position corresponding to positions 53 of the mature polypeptide consisting of SEQ ID NO:2 and an additional deletion of one or more amino acids selected from the group consisting of Ser, Glu, Thr, Asn or Pro in the loop corresponding to positions 54, 55, 56 or 57 of the mature polypeptide consisting of SEQ ID NO: 2, wherein the variant has at least 95% identity to SEQ ID NO: 2. Another embodiment concerns a method for producing a detergent composition which comprises the step of adding a protease variant which was obtained by a method comprising introducing into a parent subtilase a deletion at one positions corresponding to positions 53 of the mature polypeptide consisting of SEQ ID NO: 2, especially a method as described above, wherein the parent subtilase is selected from the group consisting of:

a. a polypeptide having at least 95% sequence identity to the mature polypeptide consisting of SEQ ID NO: 2;
b. a polypeptide encoded by a polynucleotide that hybridizes under medium or high stringency conditions with (i) the mature polypeptide coding sequence consisting of nucleotides 322 to 1146 of SEQ ID NO: 1,

(ii) a sequence encoding the mature polypeptide consisting of SEQ ID NO: 2, or (iii) the full-length complement of (i) or (ii);

c. a polypeptide encoded by a polynucleotide having at least 70% identity to the mature polypeptide coding sequence consisting of nucleotides 322 to 1146 of SEQ ID NO: 1- or a sequence encoding the mature polypeptide consisting of SEQ ID NO: 2; and
d. a fragment of the mature polypeptide consisting of SEQ ID NO: 2, which has protease activity.

A particularly preferred embodiment concerns a method for producing a detergent composition which comprises the step of adding a protease variant which was obtained by a method comprising introducing into a parent subtilase a deletion at one positions corresponding to positions 53 of the mature polypeptide consisting of SEQ ID NO: 2, wherein the variant has at least 95% identity to SEQ ID NO: 2 and wherein the method comprises deletion of one or more amino acid selected from the group consisting of Ser, Glu, Thr, Asn or Pro respectively in the loop corresponding to positions 54, 55, 56 or 57 of the mature polypeptide consisting of SEQ ID NO: 2, wherein the variant has at least 95% identity, at least 96%, at least 97%, at least 98%, or at least 99%, but less than 100%, sequence identity to the mature polypeptide consisting of SEQ ID NO: 2.

One aspect of the disclosure relates to a method for producing a detergent composition which comprises the step of adding a protease variant which was obtained by a method comprising deleting an amino acid corresponding to positions 53 of the mature polypeptide consisting of SEQ ID NO: 2, and further comprising a substitution at one or more positions corresponding to positions 53, 54, 55, 56 or 57, wherein

(a) the variant has a sequence identity to SEQ ID NO: 2 of at least 95% and less than 100% and
(b) the variant has protease activity.

In general, the protease variants according to the disclosure are preferably Subtilase variants and particularly preferred Subtilisin variants. Thus the disclosure concerns detergent compositions comprising protease variants wherein the loop comprising the positions corresponding to positions 53, 54, 55, 56 or 57 of the mature polypeptide consisting of SEQ ID NO: 2 has been shortened by at least one amino acid. In addition to deleting an amino acid in the loop corresponding to positions 53, 54, 55, 56 or 57 of the mature polypeptide consisting of SEQ ID NO: 2 also substitutions in the loop region resulted in a significantly improved wash performance compared to a protease having the identical amino acid sequence of said variant but not having the alterations at one or more of said specified positions or compared to a protease with SEQ ID NO: 2. The amino acids corresponding to positions 53-57 of SEQ ID NO: 2 form part of a loop, which connects a $\beta$-sheet with the $\alpha$-helix containing the active site residue histidine at position 64. Without being bound by any theory it is believed that altering the loop affects the active site histidine. Loop alterations that spread to the active site residue can be especially deletions, but also substitutions may have a strong enough effect to spread about 7 to 11 positions downstream in the sequence. Even subtle changes in positioning of active site residues can have significant effects on enzyme activity and thus enzyme performance. Substitutions of amino acids in the loop were done in particular with Gly, Ala, Ser, Thr and Asn, because they are small and thus do not have other unwanted effects

on the protein, e.g. steric hindrance. Furthermore Gly, Ala, Ser, Thr and Asn are not very hydrophobic, which is important at this water exposed positions.

In another aspect, the variant comprises or consists of an alteration at positions corresponding to positions 53 and 54, such as those described above. In another aspect, the variant comprises or consists of alterations at positions corresponding to positions 53 and 55, such as those described above. In another aspect, the variant comprises or consists of alterations at positions corresponding to positions 53 and 56, such as those described above.

In another aspect, the variant comprises or consists of alterations at positions corresponding to positions 53 and 57, such as those described above.

In another aspect, the variant comprises or consists of alterations at positions corresponding to positions 53, 54, and 55, such as those described above.

In another aspect, the variant comprises or consists of alterations at positions corresponding to positions 53, 54, and 56, such as those described above.

In another aspect, the variant comprises or consists of alterations at positions corresponding to positions 53, 54, and 57, such as those described above.

In another aspect, the variant comprises or consists of alterations at positions corresponding to positions 53, 55, and 56, such as those described above.

In another aspect, the variant comprises or consists of alterations at positions corresponding to positions 53, 55, and 57, such as those described above.

In another aspect, the variant comprises or consists of alterations at positions corresponding to positions 53, 56, and 57, such as those described above.

In another aspect, the variant comprises or consists of alterations at positions corresponding to positions 53, 54, 55, and 56, such as those described above.

In another aspect, the variant comprises or consists of the substitution E54A and the deletion 53* of the mature polypeptide of SEQ ID NO: 2.

In another aspect, the variant comprises or consists of the substitution T55S and the deletion 53* of the mature polypeptide of SEQ ID NO: 2.

In another aspect, the variant comprises or consists of the substitution N56A and the deletion 53* of the mature polypeptide of SEQ ID NO: 2.

In another aspect, the variant comprises or consists of the substitution P57A and the deletion 53* of the mature polypeptide of SEQ ID NO: 2.

In another aspect, the variant comprises or consists of the substitutions E54A + T55S and the deletion 53* of the mature polypeptide of SEQ ID NO: 2.

In another aspect, the variant comprises or consists of the substitutions E54A + N56A and the deletion 53* of the mature polypeptide of SEQ ID NO: 2.

In another aspect, the variant comprises or consists of the substitutions E54A + P57A and the deletion 53* of the mature polypeptide of SEQ ID NO: 2.

In another aspect, the variant comprises or consists of the substitutions T55S + N56A and the deletion 53* of the mature polypeptide of SEQ ID NO: 2.

In another aspect, the variant comprises or consists of the substitutions T55S + P57A and the deletion 53* of the mature polypeptide of SEQ ID NO: 2.

In another aspect, the variant comprises or consists of the substitutions N56A + P57A and the deletion 53* of the mature polypeptide of SEQ ID NO: 2.

In another aspect, the variant comprises or consists of the substitutions E54A + T55S + N56A and the deletion 53* of the mature polypeptide of SEQ ID NO: 2.

In another aspect, the variant comprises or consists of the substitutions E54A + T55S + P57A and the deletion 53* of the mature polypeptide of SEQ ID NO: 2.

In another aspect, the variant comprises or consists of the substitutions T55S + N56A + P57A and the deletion 53* of the mature polypeptide of SEQ ID NO: 2.

In another aspect, the variant comprises or consists of the deletions 53* + 54* of the mature polypeptide of SEQ ID NO: 2.

In another aspect, the variant comprises or consists of the deletions 53* + 55* of the mature polypeptide of SEQ ID NO: 2.

In another aspect, the variant comprises or consists of the deletions 53* + 56* of the mature polypeptide of SEQ ID NO: 2.

In another aspect, the variant comprises or consists of the deletions 53* + 57* of the mature polypeptide of SEQ ID NO: 2.

In another aspect, the variant comprises or consists of the substitutions E54A + T55S + N56A + P57A and the deletion 53* of the mature polypeptide of SEQ ID NO: 2.

The variants may further comprise one or more additional alterations at one or more (*e.g.*, several) other positions.

The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Asn/Gln, Thr/Ser, Ala/Gly, Ala/Thr, Ser/Asn, Ala/Val, Ser/Gly, Tyr/Phe, Ala/Pro, Lys/Arg, Asp/Asn, Glu/Gln, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

For example, the variants may comprise an alteration at a position corresponding to positions 54, 55, 56, 57 and further comprises an alteration at any of the positions selected from the group consisting of positions 4, 14, 63, 79, 84, 86, 88, 92, 98, 101, 146 and 217, preferably position 63 and 217 (numbering according to SEQ ID NO: 2). In a preferred embodiment the alteration at any of the positions selected from the group consisting of 4, 14, 63, 79, 84, 86, 88, 92, 98, 101, 146 and 217 is a substitution. In a particular preferred embodiment the variants in a detergent according to the invention comprise an alteration at a position corresponding to positions 53, 54, 55, 56, 57 of SEQ ID NO: 2, wherein at least one of the alterations is a deletion and wherein the variant further comprises one or more substitution selected from the group consisting of V4I, P14T, S63G, I79T, P86H, A88V, A92S, A98T, S101L, G146S or Y217L.

In a particularly preferred embodiment the variants in a detergent according to the invention further comprise the substitution Y217L.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for protease activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide. For BPN' (SEQ ID NO: 2) the catalytic triad comprising the amino acids S221, H64, and D32 is essential for protease activity of the enzyme.

The variants may consist of 200 to 900 amino acids, *e.g.*, 210 to 800, 220 to 700, 230 to 600, 240 to 500, 250 to 400, 255 to 300, 260 to 290, 265 to 285, 270 to 280 or 270, 271, 272, 273, 274, 275, 276, 277, 278, 279 or 280 amino acids.

In an embodiment, the variant has improved catalytic activity compared to the parent enzyme or compared to a protease having the identical amino acid sequence of said variant but not having the alterations at one or more of said specified positions or compared to a protease with SEQ ID NO: 2.

In an embodiment, the variant has improved wash performance compared to the parent enzyme or compared to a protease having the identical amino acid sequence of said variant but not having the alterations at one or more of said specified positions or compared to a protease with SEQ ID NO: 2, wherein wash performance is measured in AMSA as described in "Material and Methods" herein. In another embodiment, the detergent composition comprising the variant has an improved wash performance compared to an otherwise identical detergent composition with a protease being the parent subtilase or with a protease having the identical amino acid sequence of said variant but not having the alterations at one or more of said specified positions, or with a protease with SEQ ID NO: 2.

In an embodiment, a variant in a detergent according to the invention has improved thermostability compared to the parent enzyme or compared to a protease having the identical amino acid sequence of said variant but not having the alterations at one or more of said specified positions or compared to a protease with SEQ ID NO: 2, wherein the variant displaying an altered temperature-dependent activity profile at a specific temperature relative to the temperature-dependent activity profile of the parent or relative to a protease having the identical amino acid sequence of said variant but not having the alterations at one or more of said specified positions or relative to a protease with SEQ ID NO: 2. In one particular embodiment a variant in a detergent according to the invention has reduced thermal activity, wherein said

variant enhances an enzymatic reaction at a temperature lower than the temperature optimum of the parent defined by the temperature-dependent activity profile of the parent. In one particular embodiment the parent is a protease with SEQ ID NO: 2 or having at least 95 % identity hereto.

**Parent proteases**

Enzymes cleaving the amide linkages in protein substrates are classified as proteases, or (interchangeably) peptidases (see Walsh, 1979, Enzymatic Reaction Mechanisms. W.H. Freeman and Company, San Francisco, Chapter 3).

Numbering of amino acid positions/residues

If nothing else is mentioned the amino acid numbering used herein correspond to that of the subtilase BPN' (BASBPN) sequence. For further description of the BPN' sequence, see SEQ ID NO: 2 or Siezen et al., Protein Engng. 4 (1991) 719-737.

Serine proteases

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272).

The bacterial serine proteases have molecular weights in the 20,000 to 45,000 Dalton range. They are inhibited by diisopropylfluorophosphate. They hydrolyze simple terminal esters and are similar in activity to eukaryotic chymotrypsin, also a serine protease. A more narrow term, alkaline protease, covering a sub-group, reflects the high pH optimum of some of the serine proteases, from pH 9.0 to 11.0 (for review, see Priest (1977) Bacteriological Rev. 41 711-753).

Subtilases

A sub-group of the serine proteases tentatively designated subtilases has been proposed by Siezen et al., Protein Engng. 4 (1991) 719-737 and Siezen et al. Protein Science 6 (1997) 501-523. They are defined by homology analysis of more than 170 amino acid sequences of serine proteases previously referred to as subtilisin-like proteases. A subtilisin was previously often defined as a serine protease produced by Gram-positive bacteria or fungi, and according to Siezen et al. now is a subgroup of the subtilases. A wide variety of subtilases have been identified, and the amino acid sequence of a number of subtilases has been determined. For a more detailed description of such subtilases and their amino acid sequences reference is made to Siezen et al. (1997).

One subgroup of the subtilases, I-S1 or "true" subtilisins, comprises the "classical" subtilisins, such as subtilisin 168 (BSS168), subtilisin BPN', subtilisin Carlsberg (ALCALASE®, NOVOZYMES A/S), and subtilisin DY (BSSDY). BPN' is subtilisin BPN' from B. amyloliquefaciens BPN' has the amino acid sequence SEQ ID NO: 2.

A further subgroup of the subtilases, I-S2 or high alkaline subtilisins, is recognized by Siezen et al. (supra). Sub-group I-S2 proteases are described as highly alkaline subtilisins and comprises enzymes such as subtilisin PB92 (BAALKP) (MAXACAL®, DuPont/Genencor International Inc.), subtilisin 309 (SAVINASE®, NOVOZYMES A/S), subtilisin 147 (BLS147) (ESPERASE®, NOVOZYMES A/S), and alkaline elastase YaB (BSEYAB).

Subtilisins

Subtilisins are serine proteases from the family S8, in particular from the subfamily S8A, as defined by the MEROPS database (http://merops.sanger.ac.uk/cgi-bin/famsum?family=S8).
BPN' and Savinase have the MEROPS numbers S08.034 and S08.003, respectively.

**Parent subtilase**

The parent protease used in the invention is a parent subtilase. The term "parent subtilase" describes a subtilase defined according to Siezen et al. (1991 and 1997). For further details see description of "Subtilases" above. A parent subtilase may also be a subtilase isolated from a natural source, wherein subsequent modifications have been made while retaining the characteristic of a subtilase. Furthermore, a parent subtilase may be a subtilase which has been prepared by the DNA shuffling technique, such as described by J.E. Ness et al., Nature Biotechnology, 17, 893-896 (1999).

Alternatively the term "parent subtilase" may be termed "wild type subtilase".

For reference a table of the acronyms for various subtilases mentioned herein is provided, for further acronyms,

see Siezen et al., Protein Engng. 4 (1991) 719-737 and Siezen et al. Protein Science 6 (1997) 501-523.

**Table III**

| Organism | enzyme | acronym |
|---|---|---|
| **Bacteria: Gram-positive** | | |
| *Bacillus subtilis* 168 | subtilisin I168,apr | BSS168 |
| *Bacillus amyloliquefaciens* | subtilisin BPN' (NOVO) | BASBPN |
| *Bacillus subtilis DY* | subtilisin DY | BSSDY |
| *Bacillus licheniformis* | subtilisin Carlsberg | BLSCAR |
| *Bacillus lentus* | subtilisin 309 | BLSAVI |
| *Bacillus lentus* | subtilisin 147 | BLS147 |
| *Bacillus alcalophilus* PB92 | subtilisin PB92 | BAPB92 |
| *Bacillus* YaB | alkaline elastase YaB | BYSYAB |
| *Bacillus sp* NKS-21 | subtilisin ALP I | BSAPRQ |
| *Bacillus sp* G-825-6 | subtilisin Sendai | BSAPRS |
| *Thermoactinomyces vulgaris* | thermitase | TVTHER |

Modification(s) of a subtilase

The term "modification(s)" used herein is defined to include chemical modification of a subtilase as well as genetic manipulation of the DNA encoding a subtilase. The modification(s) can be replacement(s) of the amino acid side chain(s), substitution(s), deletion(s) and/or insertion(s) in or at the amino acid(s) of interest.

Subtilase variant

The term "variant" and the term "subtilase variant" are defined above.

Homologous subtilase sequences

The homology between two amino acid sequences is in this context described by the parameter "identity" for purposes of the present invention, the degree of identity between two amino acid sequences is determined using the Needleman-Wunsch algorithm as described above. The output from the routine is besides the amino acid alignment the calculation of the "Percent Identity" between the two sequences.

Based on this description it is routine for a person skilled in the art to identify suitable homologous subtilases, which can be modified according to the invention.

Substantially homologous parent subtilase variants may have one or more (several) amino acid substitutions, deletions and/or insertions, in the present context the term "one or more" is used interchangeably with the term "several". These changes are preferably of a minor nature, that is conservative amino acid substitutions as described above and other substitutions that do not significantly affect the three-dimensional folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly-histidine tract, or protein A (Nilsson et al., 1985, EMBO J. 4: 1075; Nilsson et al., 1991, Methods Enzymol. 198: 3. See, also, in general, Ford et al., 1991, Protein Expression and Purification 2: 95-107.

Although the changes described above preferably are of a minor nature, such changes may also be of a substantive nature such as fusion of larger polypeptides of up to 300 amino acids or more both as amino- or carboxyl-terminal extensions.

The parent subtilase may comprise or consist of the amino acid sequence of SEQ ID NO: 2 or an allelic variant thereof; or a fragment thereof having protease activity. In one aspect, the parent subtilase comprises or consists of the amino acid sequence of SEQ ID NO:2.

The parent subtilase may be (a) a polypeptide having at least 95% sequence identity to the mature polypeptide consisting of SEQ ID NO: 2; (b) a polypeptide encoded by a polynucleotide that hybridizes under medium or high stringency conditions with (i) the mature polypeptide coding sequence consisting of SEQ ID NO: 1, (ii) a sequence encoding the mature polypeptide consisting of SEQ ID NO: 2, or (iii) the full-length complement of (i) or (ii); or (c) a polypeptide encoded by a polynucleotide having at least 70% sequence identity to the mature polypeptide coding sequence of SEQ ID NO: 1. In an aspect, the parent has a sequence identity to the mature polypeptide of SEQ ID NO: 2

of at least 95% identity, at least 96%, at least 97%, at least 98%, or at least 99%, or 100%, which have protease activity.

In another aspect, the parent comprises or consists of the amino acid sequence of SEQ ID NO: 2. In another aspect, the parent comprises or consists of the mature polypeptide consisting of SEQ ID NO: 2. In another aspect, the parent comprises or consists of amino acids 1 to 275 of SEQ ID NO: 2.

In another aspect, the parent is encoded by a polynucleotide that hybridizes under very low stringency conditions, low stringency conditions, medium stringency conditions, or high stringency conditions, or very high stringency conditions with (i) the mature polypeptide coding sequence of SEQ ID NO: 1, (ii) a sequence encoding the mature polypeptide of SEQ ID NO: 2, or (iii) the full-length complement of (i) or (ii), (Sambrook et al., 1989, Molecular Cloning, A Laboratory Manual, 2d edition, Cold Spring Harbor, New York).

The polypeptide may be a hybrid polypeptide in which a region of one polypeptide is fused at the N-terminus or the C-terminus of a region of another polypeptide.

The parent may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

The parent may be obtained from organisms of any genus. For purposes of the present invention, the term "obtained from" as used herein in connection with a given source shall mean that the parent encoded by a polynucleotide is produced by the source or by a strain in which the polynucleotide from the source has been inserted. In one aspect, the parent is secreted extracellularly.

The parent may be a bacterial protease. For example, the parent may be a Gram-positive bacterial polypeptide such as a *Bacillus, Clostridium, Enterococcus, Geobacillus, Lactobacillus, Lactococcus, Oceanobacillus, Staphylococcus, Streptococcus,* or *Streptomyces* protease, or a Gram-negative bacterial polypeptide such as a *Campylobacter, E. coli, Flavobacterium, Fusobacterium, Helicobacter, Ilyobacter, Neisseria, Pseudomonas, Salmonella,* or *Ureaplasma* protease.

In one aspect, the parent is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* protease

In one aspect, the parent is a *Bacillus amyloliquefaciens* protease, e.g., the protease of SEQ ID NO: 2.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The parent may be identified and obtained from other sources including microorganisms isolated from nature (*e.g.,* soil, composts, water, etc.) or DNA samples obtained directly from natural materials (*e.g.,* soil, composts, water, etc.) using the above-mentioned probes. Techniques for isolating microorganisms and DNA directly from natural habitats are well known in the art. A polynucleotide encoding a parent may then be obtained by similarly screening a genomic DNA or cDNA library of another microorganism or mixed DNA sample. Once a polynucleotide encoding a parent has been detected with the probe(s), the polynucleotide can be isolated or cloned by utilizing techniques that are known to those of ordinary skill in the art (see, *e.g.,* Sambrook *et al.,* 1989, *supra*).

**Preparation of Variants**

The variants can be prepared using any mutagenesis procedure known in the art, such as site-directed mutagenesis, synthetic gene construction, semi-synthetic gene construction, random mutagenesis, shuffling, etc.

Site-directed mutagenesis is a technique in which one or more (*e.g.,* several) mutations are introduced at one or more defined sites in a polynucleotide encoding the parent.

Site-directed mutagenesis can be accomplished *in vitro* by PCR involving the use of oligonucleotide primers containing the desired mutation. Site-directed mutagenesis can also be performed *in vitro* by cassette mutagenesis

involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, *e.g.,* Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.,* U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo*, while other regions may be amplified using site-specific mutagenic primers, while yet other regions may be subjected to error-prone PCR or non-error prone PCR amplification. Polynucleotide subsequences may then be shuffled.

**Further characterization of detergent compositions according to the invention**

According to the disclosure it has surprisingly been found that detergent compositions as described above, i.e. detergent compositions comprising a protease variant as described, exhibit an advantageous wash performance, preferably an improved wash performance, especially with regard to protease-sensitive stains.

The detergent composition of the present invention may be formulated, for example, as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the present disclosure provides a detergent additive comprising a polypeptide of the present disclosure as described herein.

In a further preferred embodiment, the detergent composition according to the invention comprises the protease variant in an amount from 1 x $10^{-8}$-10 weight (wt.) percent, from 0,00001-2 wt.%, from 0,001-1 wt.%, from 0,007-0,8 wt.%, from 0,025-0,5 Gew.-% and particularly preferred from 0,04-0,38 wt.-%, based on total protein content of the protease variant. The protein concentration can be determined using known methods, for example the BCA Process (bicinchoninic acid; 2,2'-biquinolyl-4,4'-dicarboxylic acid) or the biuret process (A. G. Gornall, C. S. Bardawill and M.M. David, J. Biol. Chem., 177 (1948), pp. 751-766).

In another preferred embodiment, the detergent composition according to the invention is a liquid detergent composition.

In a further preferred embodiment, the detergent composition is characterized in that it exhibits an improved wash performance. Preferably, said improved wash performance is determined using the reference washing test as described below and as indicated in the definitions herein.

Reference washing test:

In the reference washing test, the wash performance is determined in a washing system that contains a detergent

composition at a dosing ratio of between 4.5 and 7.0 grams per liter of washing liquor as well as the protease. The detergent compositions to be compared contain the same amount of the respective protease based on total protein content, preferably providing for an amount of 5 mg protease per liter wash liquor. The wash performance is determined with respect to one or more of the following stains: chocolate milk and soot on cotton, blood, milk, ink on cotton, chocolate milk and soot on polyester/cotton, blood, milk, ink on polyester/cotton, grass on cotton, in particular with respect to one or more of the following stains:

- chocolate milk and soot on cotton: product no. C-03 obtainable from CFT (Center for Testmaterials) B.V., Vlaardingen, Netherlands,
- blood, milk, ink on cotton: product no. C-05 obtainable from CFT (Center for Testmaterials) B.V., Vlaardingen, Netherlands,
- chocolate milk and soot on polyester/cotton: product no. PC-03 obtainable from CFT (Center for Testmaterials) B.V., Vlaardingen, Netherlands,
- blood, milk, ink on polyester/cotton: product no. PC-05 obtainable from CFT (Center for Testmaterials) B.V., Vlaardingen, Netherlands,
- grass on cotton: product no. 164 obtainable from Eidgenössische Material- und Prüfanstalt (EMPA) Testmaterialien AG [Federal materials and testing agency, Testmaterials], St. Gallen, Switzerland

by measuring the whiteness of the washed textiles, the washing procedure being performed for at least 30 minutes, optionally 60 minutes, at a temperature of 40°C, and the water having a water hardness between 15.5 and 16.5° (German degrees of hardness).

The preferred liquid detergent composition for said washing system has the following composition (all indications in percentage by weight): 0.3 to 0.5% xanthan gum, 0.2 to 0.4% antifoaming agent, 6 to 7% glycerol, 0.3 to 0.5% ethanol, 4 to 7% FAEOS (fatty alcohol ether sulfate), 24 to 28% nonionic surfactants, 1% boric acid, 1 to 2% sodium citrate (dihydrate), 2 to 4% soda, 14 to 16% coconut fatty acid, 0.5% HEDP (1-hydroxyethane-(1,1-diphosphonic acid)), 0 to 0.4% PVP (polyvinylpyrrolidone), 0 to 0.05% optical brighteners, 0 to 0.001% dye, remainder deionized water. The dosing ratio of the liquid washing agent is preferably between 4.5 and 6.0 grams per liter of washing liquor, for example 4.7, 4.9, or 5.9 grams per liter of washing liquor. Washing preferably occurs in a pH range between pH 8 and pH 10.5, preferably between pH 8 and pH 9.

The preferred powdered detergent composition for said washing system has the following composition (all indications in percentage by weight): 10% linear alkylbenzenesulfonate (sodium salt), 1.5% C12 to C18 fatty alcohol sulfate (sodium salt), 2.0% C12 to C18 fatty alcohol with 7 EO, 20% sodium carbonate, 6.5% sodium hydrogencarbonate, 4.0% amorphous sodium disilicate, 17% sodium carbonate peroxohydrate, 4.0% TAED, 3.0% polyacrylate, 1.0% carboxymethyl cellulose, 1.0% phosphonate, 25% sodium sulfate; remainder: optionally foam inhibitors, optical brighteners, scents, and if applicable water to make 100%. The dosing ratio of the powdered washing agent is preferably between 5.5 and 7.0 grams per liter of washing liquor, for example 5.6, 5.9, or 6.7 grams per liter of washing liquor. Washing preferably occurs in a pH range between pH 9 and pH 11.

It is preferred according to the present invention if the aforementioned liquid washing agent is used, as indicated, to determine the wash performance.

The whiteness, i.e. the brightening of the stains, is determined as an indication of wash performance, preferably using optical measurement methods, preferably photometrically. A device suitable for this is, for example, the Minolta CM508d spectrometer. The devices used for measurement are usually calibrated beforehand using a white standard, preferably a white standard provided with the unit.

The enzyme(s) of the detergent compositions of the invention, especially the protease variants described herein, may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in, for example, WO92/19709 and WO92/19708 or the variants according to the invention may be stabilized using peptide aldehydes or ketones such as described in WO2005/105826 and WO2009/118375.

A detergent compositions according to the invention might further comprise one or more peroxy compounds.

Such peroxy compounds optionally present in the compositions which may in particular be considered are organic peracids or peracidic salts of organic acids, such as phthalimidopercaproic acid, perbenzoic acid or salts of diperdodecanedioic acid, hydrogen peroxide and inorganic salts which release hydrogen peroxide under the washing conditions, such as perborate, percarbonate and/or persilicate. Hydrogen peroxide may here also be produced with the assistance of an enzymatic system, i.e. an oxidase and its substrate. Where solid peroxy compounds are to be used, they may be used in the form of powders or granules, which may also in principle be encapsulated in known manner. Alkali metal percarbonate, alkali metal perborate monohydrate, alkali metal perborate tetrahydrate or hydrogen peroxide in the form of aqueous solutions containing 3wt.% to 10 wt.% of hydrogen peroxide are particularly preferred. Peroxy

compounds are preferably present in washing or cleaning agents according to the invention in quantities of up to 50 wt.%, in particular of 5 wt.% to 30 wt.%.

Apart from the protease variant to be used according to the invention, the detergent compositions according to the invention, which may in particular take the form of pulverulent solids, post-compressed particles, homogeneous solutions or suspensions, may in principle contain any ingredients known and conventional in such compositions. The compositions according to the invention may in particular contain builder substances, surface-active surfactants, water-miscible organic solvents, enzymes, sequestering agents, electrolytes, pH regulators, polymers with specific effects, such as soil release polymers, dye transfer inhibitors, graying inhibitors, crease-reducing polymeric active ingredients and shape-retaining polymeric active ingredients, and further auxiliary substances, such as optical brighteners, foam regulators, additional peroxy activators, colorants and scents.

In addition to the above-stated ingredients, a composition according to the invention may contain conventional antimicrobial active ingredients in order to enhance the disinfection action, for example towards specific microorganisms. Such antimicrobial additives are preferably present in the disinfectants according to the invention in quantities of up to 10 wt.%, in particular of 0.1 wt.% to 5 wt.%.

In a detergent composition according to the invention, conventional bleach activators which form peroxycarboxylic acid or peroxyimidic acids under perhydrolysis conditions and/or conventional bleach-activating transition metal complexes may also be used. The bleach activator component optionally present in particular in quantities of 0.5 wt.% to 6 wt.% comprises conventionally used Nor O-acyl compounds, for example polyacylated alkylenediamines, in particular tetraacetylethylenediamine, acylated glycolurils, in particular tetraacetylglycoluril, N-acylated hydantoins, hydrazides, triazoles, urazoles, diketopiperazines, sulfurylamides and cyanurates, moreover carboxylic anhydrides, in particular phthalic anhydride, carboxylic acid esters, in particular sodium isononanoylphenolsulfonate, and acylated sugar derivatives, in particular pentaacetyl glucose, together with cationic nitrile derivatives such as trimethylammonium acetonitrile salts. In order to avoid interaction with per compounds during storage, the bleach activators may in known manner have been coated with shell substances or granulated, particularly preferred options being tetraacetylethylenediamine granulated with the assistance of carboxymethylcellulose and having an average grain size of 0.01 mm to 0.8 mm, granulated 1,5-diacetyl-2,4-dioxohexahydro-1,3,5-triazine, and/or trialkylammonium acetonitrile formulated in particulate form. Such bleach activators are preferably present in washing or cleaning compositions in quantities of up to 8 wt.%, in particular of 2wt.% to 6 wt.%, in each case relative to entire composition.

The compositions according to the invention may contain one or more surfactants, with anionic surfactants, nonionic surfactants and mixtures thereof in particular being considered, but cationic and/or amphoteric surfactants also possibly being present. Suitable nonionic surfactants are in particular alkyl glycosides and ethoxylation and/or propoxylation products of alkyl glycosides or linear or branched alcohols in each case having 12 to 18 C atoms in the alkyl moiety and 3 to 20, preferably 4 to 10, alkyl ether groups. Corresponding ethoxylation and/or propoxylation products of N-alkylamines, vicinal diols, fatty acid esters and fatty acid amides, which correspond with regard to the alkyl moiety to the stated long-chain alcohol derivatives, and of alkylphenols having 5 to 12 C atoms in the alkyl residue may furthermore be used.

Suitable anionic surfactants are in particular soaps and those which contain sulfate or sulfonate groups with preferably alkali metal ions as cations. Usable soaps are preferably the alkali metal salts of saturated or unsaturated fatty acids with 12 to 18 C atoms. Such fatty acids may also be used in incompletely neutralized form. Usable surfactants of the sulfate type include the salts of sulfuric acid semiesters of fatty alcohols with 12 to 18 C atoms and the sulfation products of the stated nonionic surfactants with a low degree of ethoxylation. Usable surfactants of the sulfonate type include linear alkylbenzene sulfonates with 9 to 14 C atoms in the alkyl moiety, alkane-sulfonates with 12 to 18 C atoms, and olefin sulfonates with 12 to 18 C atoms, which arise from the reaction of corresponding monoolefins with sulfur trioxide, and alpha-sulfofatty acid esters which arise from the sulfonation of fatty acid methyl or ethyl esters.

Such surfactants are present in the washing compositions according to the invention in proportions of preferably 5 wt.% to 50 wt.%, in particular of 8 wt.% to 30 wt.%, while the disinfectants according to the invention and the cleaning compositions according to the invention preferably contain 0.1 wt.% to 20 wt.%, in particular 0.2 wt.% to 5 wt.% of surfactants.

The compositions according to the invention, in particular if they are compositions intended for treating textiles, may in particular contain one or more of the cationic, textile-softening substances of the general formulae X, XI, or XII as cationic active substances with a textile-softening action:

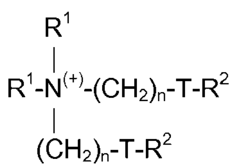

(X)

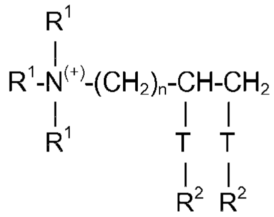

(XI)

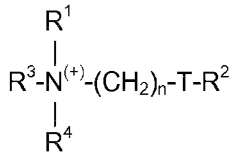

(XII)

in which each group $R^1$ is mutually independently selected from among $C_{1-6}$ alkyl, alkenyl or hydroxyalkyl groups; each group $R^2$ is mutually independently selected from among $C_{8-28}$ alkyl or alkenyl groups; $R^3 = R^1$ or $(CH_2)_n$-T-$R^2$; $R^4 = R^1$ or $R^2$ or $(CH_2)_n$-T-$R^2$; T = $-CH_2-$, -O-CO- or -CO-O- and n is an integer from 0 to 5. The cationic surfactants comprise conventional anions of a nature and number required for charge balancing, it being possible to select said anions not only from, for example, halides but also from anionic surfactants. In preferred embodiments of the present invention, cationic surfactants which may be used are hydroxyalkyltrialkylammonium compounds, in particular $C_{12-18}$ alkyl(hydroxyethyl)dimethylammonium compounds, and preferably the halides thereof, in particular chlorides. A composition according to the invention preferably contains 0.5 wt.% to 25 wt.%, in particular 1 wt.% to 15 wt.% of cationic surfactant.

A composition according to the invention preferably contains at least one water-soluble and/or water-insoluble, organic and/or inorganic builder. Water-soluble organic builder substances include polycarboxylic acids, in particular citric acid and saccharic acids, monomeric and polymeric aminopolycarboxylic acids, in particular methylglycinediacetic acid, nitrilotriacetic acid and ethylenediaminetetraacetic acid together with polyaspartic acid, polyphosphonic acids, in particular aminotris(methylenephosphonic acid), ethylenediamine-tetrakis(methylenephosphonic acid) and 1-hydroxyethane-1,1-diphosphonic acid, polymeric hydroxyl compounds such as dextrin and polymeric (poly-)carboxylic acids, in particular polycarboxylates obtainable by oxidizing polysaccharides or dextrins, and/or polymeric acrylic acids, methacrylic acids, maleic acids and copolymers thereof, which may also contain small proportions of polymerizable substances without carboxylic acid functionality. The relative molecular mass of the homopolymers of unsaturated carboxylic acids is in general between 5,000 and 200,000, that of the copolymers between 2,000 and 200,000, preferably 50,000 to 120,000, in each case relative to free acid. One particularly preferred acrylic acid/maleic acid copolymer has a relative molecular mass of 50,000 to 100,000. Suitable, albeit less preferred, compounds of this class are copolymers of acrylic acid or methacrylic acid with vinyl ethers, such as vinyl methyl ethers, vinyl esters, ethylene, propylene and styrene, the acid fraction of which amounts to at least 50 wt.%. Terpolymers containing as monomers two unsaturated acids and/or the salts thereof and, as third monomer, vinyl alcohol and/or an esterified vinyl alcohol or a carbohydrate may also be used as water-soluble organic builder substances. The first acidic monomer or the salt thereof is derived from a monoethylenically unsaturated $C_3$-$C_8$ carboxylic acid and preferably from a $C_3$-$C_4$ monocarboxylic acid, in particular from (meth)acrylic acid. The second acidic monomer or the salt thereof may be a derivative of a $C_4$-$C_8$ dicarboxylic acid, maleic acid being particularly preferred, and/or a derivative of an allylsulfonic acid which is substituted in position 2 with an alkyl or aryl residue. Such polymers generally have a relative molecular mass of between 1,000 and 200,000. Further preferred copolymers are those which comprise acrolein and acrylic acid/acrylic acid salts or vinyl acetate as monomers. The organic builder substances may be used, in particular for producing liquid compositions, in the form of aqueous solutions, preferably in the form of 30 to 50 wt.% aqueous solutions. All the stated acids are generally used in the form of the water-soluble salts, in particular the alkali metal salts, thereof.

Such organic builder substances may, if desired, be present in quantities of up to 40 wt.%, in particular of up to 25 wt.% and preferably of 1 wt.% to 8 wt.%. Quantities close to the stated upper limit are preferably used in pasty or liquid, in particular water-containing, compositions according to the invention.

Water-soluble inorganic builder materials which may in particular be considered are polymeric alkali metal phosphates, which may be present in the form of the alkaline, neutral or acidic sodium or potassium salts thereof. Examples are tetrasodium diphosphate, disodium dihydrogen diphosphate, pentasodium triphosphate, "sodium hexametaphosphate" and the corresponding potassium salts or mixtures of sodium and potassium salts. Water-insoluble, water-dispersible inorganic builder materials which are used are in particular crystalline or amorphous alkali metal aluminosilicates, in quantities of up to 50 wt.%, preferably of no more than 40 wt.% and, in liquid compositions, in particular from 1 wt.% to 5 wt.%. Among these, washing composition grade crystalline sodium aluminosilicates, in particular zeolite A, P and optionally X, are preferred. Quantities close to the stated upper limit are preferably used in solid, particulate compositions.

Suitable aluminosilicates in particular comprise no particles with a grain size of above 30 μm and preferably consist to an extent of at least 80 wt.% of particles with a size below 10 μm. Their calcium binding capacity, which may be determined as stated in German patent DE 24 12 837, is generally in the range of from 100 to 200 mg of CaO per gram.

Suitable substitutes or partial substitutes for the stated aluminosilicate are crystalline alkali metal silicates, which may be present alone or mixed with amorphous silicates. The alkali metal silicates usable as builders in the compositions according to the invention preferably have a molar ratio of alkali metal oxide to $SiO_2$ of below 0.95, in particular of 1:1.1 to 1:12 and may be in amorphous or crystalline form. Preferred alkali metal silicates are sodium silicates, in particular amorphous sodium silicates, with a molar ratio $Na_2O$:$SiO_2$ of 1:2 to 1:2.8. Preferably used crystalline silicates, which may be present alone or mixed with amorphous silicates, are crystalline phyllosilicates of the general formula $Na_2Si_xO_{2x+i} \cdot y\ H_2O$, in which x, the "modulus", is a number from 1.9 to 4 and y is a number from 0 to 20 and preferred values for x are 2, 3 or 4. Preferred crystalline phyllosilicates are those in which x in the stated general formula assumes the values 2 or 3. In particular, both β- and δ-sodium disilicates ($Na_2Si_2O_5 \cdot y\ H_2O$) are preferred. Virtually anhydrous crystalline alkali metal silicates, produced from amorphous alkali metal silicates, of the above-stated general formula in which x means a number from 1.9 to 2.1 may also be used in compositions according to the invention. A crystalline sodium phyllosilicate with a modulus of 2 to 3, as may be produced from sand and soda, is used in a further preferred embodiment of compositions according to the invention. Crystalline sodium silicates with a modulus in the range from 1.9 to 3.5 are used in a further preferred embodiment of compositions according to the invention. In one preferred development of compositions according to the invention, a granular compound of alkali metal silicate and alkali metal carbonate is used, as is for example commercially available under the name Nabion® 15. If alkali metal aluminosilicate, in particular zeolite, is present as an additional builder substance, the weight ratio of aluminosilicate to silicate, in each case relative to anhydrous active substances, preferably amounts to 1:10 to 10:1. In compositions which contain both amorphous and crystalline alkali metal silicates, the weight ratio of amorphous alkali metal silicate to crystalline alkali metal silicate preferably amounts to 1:2 to 2:1 and in particular to 1:1 to 2:1.

Builder substances are present in the washing or cleaning compositions according to the invention preferably in quantities of up to 60 wt.%, in particular of 5 wt.% to 40 wt.%.

In a preferred development of the invention, a composition according to the invention comprises a water-soluble "builder block". Use of the term "builder block" is intended to indicate that the composition does not contain any further builder substances as such which are water-soluble, i.e. all the builder substances present in the composition are combined in the "block" characterized in this manner, an exception being, however, made for the quantities of substances which may be present, as is conventional in commerce, in small quantities as contaminants or stabilizing additives in the other ingredients of the compositions. The term "water-soluble" should here be taken to mean that, at the concentration which is obtained under conventional conditions by the input quantity of the composition containing it, the builder block dissolves without leaving a residue. The compositions according to the invention preferably contain at least 15 wt.% and up to 55 wt.%, in particular 25 wt.% to 50 wt.% of water-soluble builder block. The latter is preferably composed of the components

a) 5 wt.% to 35 wt.% citric acid, alkali metal citrate and/or alkali metal carbonate, which may also at least in part be replaced by alkali metal hydrogencarbonate,
b) up to 10 wt.% alkali metal silicate with a modulus in the range from 1.8 to 2.5,
c) up to 2 wt.% phosphonic acid and/or alkali metal phosphonate,
d) up to 50 wt.% alkali metal phosphate, and
e) up to 10 wt.% polymeric polycarboxylate,

the stated quantities relating to the entire washing or cleaning composition. Unless explicitly stated otherwise, this also applies to all the quantities stated below.

In a preferred embodiment of compositions according to the invention, the water-soluble builder block contains at least 2 of components b), c), d) and e) in quantities of greater than 0 wt.%.

With regard to component a), in a preferred embodiment of compositions according to the invention, 15 wt.% to 25 wt.% of alkali metal carbonate, which may at least in part be replaced by alkali metal hydrogencarbonate, and up to 5 wt.%, in particular 0.5 wt.% to 2.5 wt.% of citric acid and/or alkali metal citrate are present. In an alternative embodiment of compositions according to the invention, 5 wt.% to 25 wt.%, in particular 5 wt.% to 15 wt.% of citric acid and/or alkali metal citrate and up to 5 wt.% , in particular 1 wt.% to 5 wt.% of alkali metal carbonate, which may at least in part be replaced by alkali metal hydrogencarbonate, are present as component a). If both alkali metal carbonate and alkali metal hydrogencarbonate are present, component a) preferably comprises alkali metal carbonate and alkali metal hydrogencarbonate in a weight ratio of 10:1 to 1:1.

With regard to component b), in a preferred embodiment of compositions according to the invention, 1 wt.% to 5 wt.% of alkali metal silicate with a modulus in the range from 1.8 to 2.5 are present.

With regard to component c), in a preferred embodiment of compositions according to the invention, 0.05 wt.%

to 1 wt.% of phosphonic acid and/or alkali metal phosphonate are present. Phosphonic acids are here also taken to mean optionally substituted alkylphosphonic acids, which may also comprise two or more phosphonic acid groups ("polyphosphonic acids"). They are preferably selected from hydroxy- and/or aminoalkylphosphonic acids and/or the alkali metal salts thereof, such as for example dimethylaminomethane diphosphonic acid, 3-aminopropyl-1-hydroxy-1,1-diphosphonic acid, 1-amino-1-phenylmethane diphosphonic acid, 1-hydroxyethane-1,1-diphosphonic acid, aminotris(methylenephosphonic acid), N,N,N',N'-ethylenediamine-tetrakis-(methylenephosphonic acid) and acylated derivatives of phosphorous acid, which may also be used in any desired mixtures.

With regard to component d), in a preferred embodiment of compositions according to the invention, 15 wt.% to 35 wt.% of alkali metal phosphate, in particular trisodium polyphosphate, are present. "Alkali metal phosphate" is the summary name for the alkali metal (in particular sodium and potassium) salts of the various phosphoric acids, it being possible to distinguish between meta-phosphoric acids $(HPO_3)_n$ and ortho-phosphoric acid $H_3PO_4$ as well as higher molecular weight representatives. The phosphates here combine a number of advantages: they act as alkalinity donors, prevent lime deposits on machine parts or lime incrustation of fabrics and, moreover, contribute to cleaning performance. Sodium dihydrogenphosphate, $NaH_2PO_4$, exists as a dihydrate (density 1.91 $gcm^{-3}$, melting point 60°C) and as a monohydrate (density 2.04 $gcm^{-3}$). Both salts are white powders, very readily soluble in water, which lose their water of crystallization when heated and at 200°C change into the weakly acidic diphosphate (disodium hydrogendiphosphate, $Na_2H_2P_2O_7$) and at a higher temperature into sodium trimetaphosphate ($Na_3P_3O_9$) and Maddrell's salt. $NaH_2PO_4$ exhibits an acidic reaction; it is obtained when phosphoric acid is adjusted with sodium hydroxide solution to a pH value of 4.5 and the slurry is atomized. Potassium dihydrogenphosphate (primary or monobasic potassium phosphate, potassium diphosphate, KDP), $KH_2PO_4$, is a white salt with a density of 2.33 $gcm^{-3}$, has a melting point of 253°C (decomposition with formation of $(KPO_3)_x$ potassium polyphosphate) and is readily soluble in water. Disodium hydrogenphosphate (secondary sodium phosphate), $Na_2HPO_4$, is a colorless, very readily water-soluble crystalline salt. It exists in anhydrous form and with 2 mol (density 2.066 $gcm^{-3}$, water loss at 95°C), 7 mol (density 1.68 $gcm^{-3}$, melting point 48°C with loss of 5 $H_2O$) and 12 mol of water (density 1.52 $gcm^{-3}$, melting point 35°C with loss of 5 $H_2O$), is anhydrous at 100°C and when heated further changes into the diphosphate $Na_4P_2O_7$. Disodium hydrogenphosphate is produced by neutralizing phosphoric acid with soda solution using phenolphthalein as indicator. Dipotassium hydrogenphosphate (secondary or dibasic potassium phosphate), $K_2HPO_4$, is an amorphous, white salt, which is readily soluble in water. Trisodium phosphate, tertiary sodium phosphate, $Na_3PO_4$, are colorless crystals, which have as dodecahydrate a density of 1.62 $gcm^{-3}$ and a melting point of 73-76°C (decomposition), as decahydrate (corresponding to 19-20% $P_2O_5$) a melting point of 100°C and in anhydrous form (corresponding to 39-40% $P_2O_5$) a density of 2.536 $gcm^{-3}$. Trisodium phosphate is readily soluble in water with an alkaline reaction and is produced by evaporation of a solution of precisely 1 mol of disodium phosphate and 1 mol of NaOH. Tripotassium phosphate (tertiary or tribasic potassium phosphate), $K_3PO_4$, is a white, deliquescent, granular powder with a density of 2.56 $gcm^{-3}$, has a melting point of 1340°C and is readily soluble in water with an alkaline reaction. It arises for example when Thomas slag is heated with carbon and potassium sulfate. Despite their relatively high price, the more readily soluble and therefore highly effective potassium phosphates are often preferred in the cleaning composition industry over corresponding sodium compounds. Tetrasodium diphosphate (sodium pyrophosphate), $Na_4P_2O_7$, exists in anhydrous form (density 2.534 $gcm^{-3}$, melting point 988°C, also stated as 880°C) and as decahydrate (density 1.815-1.836 $gcm^{-3}$, melting point 94°C with water loss). Solid substances comprise colorless crystals which are soluble in water with an alkaline reaction. $Na_4P_2O_7$ arises on heating disodium phosphate to >200°C or by reacting phosphoric acid with soda in a stoichiometric ratio and dehydrating the solution by atomization. The decahydrate complexes heavy metal salts and hardness forming substances and therefore reduces the hardness of the water. Potassium diphosphate (potassium pyrophosphate), $K_4P_2O_7$, exists in the form of the trihydrate and is a colorless, hygroscopic powder with a density of 2.33 $gcm^{-3}$, which is soluble in water, the pH value of the 1% solution amounting to 10.4 at 25°C. Condensing $NaH_2PO_4$ or $KH_2PO_4$ gives rise to higher molecular weight sodium and potassium phosphates, in which it is possible to distinguish between cyclic representatives, the sodium or potassium metaphosphates, and chain types, the sodium or potassium polyphosphates The latter in particular have a plurality of names: fused or thermal phosphates, Graham's salt, Kurrol's salt and Maddrell's salt. All higher sodium and potassium phosphates are jointly designated condensed phosphates. The technically important pentasodium triphosphate, $Na_5P_3O_{10}$ (sodium tripolyphosphate), is a non-hygroscopic, white, water-soluble salt, which is anhydrous or crystallized with 6 $H_2O$, of the general formula $NaO-[P(O)(ONa)-O]_n-Na$ with n=3. At room temperature approx. 17 g, at 60°C approx. 20 g, at 100°C around 32 g of the salt containing no water of crystallization dissolve in 100 g of water; after heating the solution for two hours to 100°C, approx. 8% orthophosphate and 15% diphosphate are obtained by hydrolysis. When producing pentasodium triphosphate, phosphoric acid is reacted with soda solution or sodium hydroxide solution in a stoichiometric ratio and the solution is dehydrated by atomization. As with Graham's salt and sodium diphosphate, pentasodium triphosphate dissolves many insoluble metal compounds (even lime soaps etc.). Pentapotassium triphosphate, $K_5P_3O_{10}$ (potassium tripolyphosphate), is commercially available, for example, in the form of a 50 wt.% solution (>23% $P_2O_5$, 25% $K_2O$). Potassium polyphosphates are widely used in the washing and cleaning composition industry. Sodium potassium tripolyphosphates also exist, which may likewise be used for the purposes of the present invention. These arise for

example if sodium trimetaphosphate is hydrolyzed with KOH:

$$(NaPO_3)_3 + 2\ KOH \rightarrow Na_3K_2P_3O_{10} + H_2O$$

They may be used in exactly the same way as sodium tripolyphosphate, potassium tripolyphosphate or mixtures of these two; mixtures of sodium tripolyphosphate and sodium potassium tripolyphosphate or mixtures of potassium tripolyphosphate and sodium potassium tripolyphosphate or mixtures of sodium tripolyphosphate and potassium tripolyphosphate and sodium potassium tripolyphosphate may also be used according to the invention.

With regard to component e), in a preferred embodiment of compositions according to the invention, 1.5 wt.% to 5 wt.% of polymeric polycarboxylate are present, in particular selected from the polymerization or copolymerization products of acrylic acid, methacrylic acid and/or maleic acid. Among these, the homopolymers of acrylic acid are preferred and, among these in turn, those with an average molar mass in the range from 5,000 D to 15,000 D (PA standard) are particularly preferred.

In another preferred embodiment, the detergent composition according to the invention comprises one or more additional enzymes such as a protease, lipase, cutinase, an amylase, carbohydrase, cellulase, pectinase, mannanase, arabinase, galactanase, xylanase, oxidase, e.g., a laccase, and/or peroxidase.

In general the properties of the selected enzyme(s) should be compatible with the selected detergent, (i.e., pH-optimum, compatibility with other enzymatic and non-enzymatic ingredients, etc.), and the enzyme(s) should be present in effective amounts.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (DuPont/Genencor International Inc.), and KAC-500(B)™ (Kao Corporation).

Proteases: The additional enzyme may be another protease or protease variant. The protease may be of animal, vegetable or microbial origin, including chemically or genetically modified mutants. Microbial origin is preferred. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4, M5, M7 or M8.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family. In one aspect of the invention the additional protease may be a subtilase, such as a subtilisin or a variant hereof.

Examples of subtilisins are those derived from Bacillus such as subtilisin lentus, Bacillus lentus, subtilisin Novo, subtilisin Carlsberg, Bacillus licheniformis, subtilisin BPN', subtilisin 309, subtilisin 147 and subtilisin 168 described in WO 89/06279 and protease PD138 (WO 93/18140). Additional serine protease examples are described in WO 98/020115, WO 01/44452, WO 01/58275, WO 01/58276, WO 03/006602 and WO 04/099401. Further examples of subtilase variants may be those having mutations in any of the positions: 3, 4, 9, 15, 27, 36, 68, 76, 87, 95, 96, 97, 98, 99, 100, 101, 102, 103, 104, 106, 118, 120, 123, 128, 129, 130, 160, 167, 170, 194, 195, 199, 205, 217, 218, 222, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering). A further preferred protease is the alkaline protease from Bacillus lentus DSM 5483, as described for example in WO 95/23221, and variants thereof which are described in WO 92/21760, WO 95/23221, EP 1921147 and EP 1921148.

Examples of trypsin-like proteases are trypsin (e.g. of porcine or bovine origin) and the Fusarium protease described in WO 89/06270 and WO 94/25583. Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235, and 274.

Examples of metalloproteases are the neutral metalloprotease as described in WO 07/044993.

Preferred commercially available protease enzymes include Alcalase™, Coronase™, Duralase™, Durazym™, Esperase™, Everlase™, Kannase™, Liquanase™, Liquanase Ultra™, Ovozyme™, Polarzyme™, Primase™, Relase™, Savinase™ and Savinase Ultra™, (Novozymes A/S), Axapem™ (Gist-Brocases N.V.), Excellase™, FN2™, FN3™, FN4™, Maxaca™, Maxapem™, Maxatase™, Properase™, Purafast™, Purafect™, Purafect OxP™, Purafect Prime™ and Puramax™ (DuPont/Genencor int.).

Lipases and Cutinases: Suitable lipases and cutinases include those of bacterial or fungal origin. Chemically modified or protein engineered mutants are included. Examples include lipase from *Thermomyces*, *e.g.,* from *T. lanuginosus* (previously named *Humicola lanuginosa*) as described in EP 258 068 and EP 305 216, cutinase from *Humicola, e.g. H. insolens* as described in WO 96/13580, a *Pseudomonas* lipase, *e.g.,* from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, *e.g.,* from *B. subtilis* (Dartois et al., 1993, Biochemica et Biophysica Acta, 1131: 253-360), B. *stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079, WO 97/07202, WO 00/060063, WO2007/087508 and WO 2009/109500.

Preferred commercially available lipase enzymes include Lipolase™, Lipolase Ultra™, and Lipex™; Lecitase™, Lipolex™; Lipoclean™, Lipoprime™ (Novozymes A/S). Other commercially available lipases include Lumafast (DuPont/Genencor Int Inc); Lipomax (Gist-Brocades/DuPont/Genencor Int Inc) and Bacillus sp lipase from Solvay.

Amylases: Suitable amylases ($\alpha$ and/or $\beta$) include those of bacterial or fungal origin. Chemically modified or protein engineered mutants are included. Amylases include, for example, $\alpha$-amylases obtained from *Bacillus, e.g.*, a special strain of *Bacillus licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novozymes A/S), Rapidase™ and Purastar™ (from DuPont/Genencor International Inc.).

Peroxidases/Oxidases: Suitable peroxidases/oxidases include those of plant, bacterial or fungal origin. Chemically modified or protein engineered mutants are included. Examples of useful peroxidases include peroxidases from *Coprinus*, *e.g.,* from C. *cinereus,* and variants thereof as those described in WO 93/24618, WO 95/10602, and WO 98/15257.

Commercially available peroxidases include Guardzyme™ (Novozymes A/S).

The detergent enzyme(s) may be included in a detergent composition by adding separate additives containing one or more enzymes, or by adding a combined additive comprising all of these enzymes. A detergent additive, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, *e.g.,* as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

In another preferred embodiment of the invention, the composition contains 5 wt.% to 50 wt.%, in particular 8-30 wt.% of anionic and/or nonionic surfactant, up to 60 wt.%, in particular 5-40 wt.% of builder substance and 0.2 wt.% to 2 wt.% of enzyme, selected from proteases, lipases, cutinases, amylases, pullulanases, mannanases, cellulases, oxidases and peroxidases and mixtures thereof.

Organic solvents which may be used in the compositions according to the invention, in particular if these are in liquid or pasty form, include alcohols with 1 to 4 C atoms, in particular methanol, ethanol, isopropanol and tert.-butanol, diols with 2 to 4 C atoms, in particular ethylene glycol and propylene glycol, and mixtures thereof and the ethers derivable from the stated classes of compounds. Such water-miscible solvents are preferably present in the washing compositions according to the invention in quantities of no more than 30 wt.%, in particular of 6 wt.% to 20 wt.%.

In order to establish a desired pH value which is not automatically obtained by mixing the remaining components, the compositions according to the invention may contain acids which are compatible with the system and are environmentally compatible, in particular citric acid, acetic acid, tartaric acid, malic acid, lactic acid, glycolic acid, succinic acid,

glutaric acid and/or adipic acid, as well as mineral acids, in particular sulfuric acid, or bases, in particular ammonium or alkali metal hydroxides. Such pH regulators are present in the compositions according to the invention preferably in an amount of no more than 20 wt.%, in particular of 1.2 wt.% to 17 wt.%.

Polymers with a soil detachment capacity, which are often known as "soil release" active ingredients or, due to their ability to provide a soil-repelling finish on the treated surface, for example the fiber, as "soil repellents," are for example nonionic or cationic cellulose derivatives. Polymers with a soil detachment capacity, in particular with regard to polyesters, include copolyesters prepared from dicarboxylic acids, for example adipic acid, phthalic acid or terephthalic acid, diols, for example ethylene glycol or propylene glycol, and polydiols, for example polyethylene glycol or polypropylene glycol. Polyesters with a soil detachment capacity which are preferably used include those compounds which, in formal terms, are obtainable by esterifying two monomer moieties, the first monomer being a dicarboxylic acid HOOC-Ph-COOH and the second monomer a diol $HO-(CHR^{11}-)_aOH$, which may also be present as a polymeric diol $H-(O-(CHR^{11}-)_a)_bOH$. Ph here means an o-, m- or p-phenylene residue which may bear 1 to 4 substituents selected from alkyl residues with 1 to 22 C atoms, sulfonic acid groups, carboxyl groups and mixtures thereof, $R^{11}$ means hydrogen, an alkyl residue with 1 to 22 C atoms and mixtures thereof, a means a number from 2 to 6 and b a number from 1 to 300. The polyesters obtainable therefrom preferably contain not only monomer diol units $-O-(CHR^{11}-)_aO-$ but also polymer diol units $-(O-(CHR^{11}-)_a)_bO-$. The molar ratio of monomer diol units to polymer diol units preferably amounts to 100:1 to 1:100, in particular to 10:1 to 1:10. In the polymer diol units, the degree of polymerization b is preferably in the range from 4 to 200, in particular from 12 to 140. The molecular weight or average molecular weight or the maximum of the molecular weight distribution of preferred polyesters with a soil detachment capacity is in the range from 250 to 100,000, in particular from 500 to 50,000. The acid on which the residue Ph is based is preferably selected from terephthalic acid, isophthalic acid, phthalic acid, trimellitic acid, mellitic acid, the isomers of sulfophthalic acid, sulfoisophthalic acid and sulfoterephthalic acid and mixtures thereof. Where the acid groups thereof are not part of the ester bond in the polymer, they are preferably present in salt form, in particular as an alkali metal or ammonium salt. Among these, sodium and potassium salts are particularly preferred. If desired, instead of the monomer HOOC-Ph-COOH, the polyester with a soil detachment capacity may contain small proportions, in particular no more than 10 mol% relative to the proportion of Ph with the above-stated meaning, of other acids which comprise at least two carboxyl groups. These include, for example, alkylene and alkenylene dicarboxylic acids such as malonic acid, succinic acid, fumaric acid, maleic acid, glutaric acid, adipic acid, pimelic acid, suberic acid, azelaic acid and sebacic acid. Preferred diols $HO-(CHR^{11}-)_aOH$ include those in which $R^{11}$ is hydrogen and a is a number from 2 to 6, and those in which a has the value 2 and $R^{11}$ is selected from hydrogen and alkyl residues with 1 to 10, in particular 1 to 3 C atoms. Among the latter-stated diols, those of the formula $HO-CH_2-CHR^{11}-OH$, in der $R^{11}$ has the above-stated meaning, are particularly preferred. Examples of diol components are ethylene glycol, 1,2-propylene glycol, 1,3-propylene glycol, 1,4-butanediol, 1,5-pentanediol, 1,6-hexanediol, 1,8-octanediol, 1,2-decanediol, 1,2-dodecanediol and neopentyl glycol. Among the polymeric diols, polyethylene glycol with an average molar mass in the range from 1000 to 6000 is particularly preferred. If desired, these polyesters may also be end group-terminated, with end groups which may be considered being alkyl groups with 1 to 22 C atoms and esters of monocarboxylic acids. The end groups attached via ester bonds may be based on alkyl, alkenyl and aryl monocarboxylic acids with 5 to 32 C atoms, in particular 5 to 18 C atoms. These include valeric acid, caproic acid, enanthic acid, caprylic acid, pelargonic acid, capric acid, undecanoic acid, undecenoic acid, lauric acid, lauroleic acid, tridecanoic acid, myristic acid, myristoleic acid, pentadecanoic acid, palmitic acid, stearic acid, petroselinic acid, petroselaidic acid, oleic acid, linoleic acid, linolaidic acid, linolenic acid, eleostearic acid, arachidic acid, gadoleic acid, arachidonic acid, behenic acid, erucic acid, brassidic acid, clupanodonic acid, lignoceric acid, cerotic acid, melissic acid, benzoic acid, which may bear 1 to 5 substituents having a total of up to 25 C atoms, in particular 1 to 12 C atoms, for example tert.-butylbenzoic acid. The end groups may also be based on hydroxymonocarboxylic acids with 5 to 22 C atoms, which for example include hydroxyvaleric acid, hydroxycaproic acid, ricinoleic acid, the hydrogenation product thereof, hydroxystearic acid, and o-, m- and p-hydroxybenzoic acid. The hydroxymonocarboxylic acids may in turn be joined to one another via their hydroxyl group and their carboxyl group and thus be repeatedly present in an end group. The number of hydroxymonocarboxylic acid units per end group, i.e. their degree of oligomerization, is preferably in the range from 1 to 50, in particular from 1 to 10. In a preferred development of the invention, polymers of ethylene terephthalate and polyethylene oxide terephthalate, in which the polyethylene glycol units have molar weights of 750 to 5000 and the molar ratio of ethylene terephthalate to polyethylene oxide terephthalate amounts to 50:50 to 90:10, are used alone or in combination with cellulose derivatives.

Dye transfer inhibitors which may be considered for use in compositions according to the invention for washing textiles include in particular polyvinylpyrrolidones, polyvinylimidazoles, polymeric N-oxides such as poly-(vinylpyridine-N-oxide) and copolymers of vinylpyrrolidone with vinylimidazole and optionally further monomers.

The compositions according to the invention for use in washing textile may contain anticrease compositions since textile fabrics, in particular made from rayon, wool, cotton and mixtures thereof, may have a tendency to crease, because the individual fibers are sensitive to being bent, kinked, pressed and squashed transversely of the fiber direction. These include for example synthetic products based on fatty acids, fatty acid esters, fatty acid amides, fatty acid alkylol

esters, fatty acid alkylol amides or fatty alcohols, which have generally been reacted with ethylene oxide, or products based on lecithin or modified phosphoric acid esters.

Graying inhibitors have the task of keeping dirt which has been dissolved away from the hard surface and in particular from the textile fiber suspended in the liquor. Water-soluble colloids of a mainly organic nature are suitable for this purpose, for example starch, size, gelatin, salts of ether carboxylic acids or ether sulfonic acids of starch or cellulose or salts of acidic sulfuric acid esters of cellulose or starch. Water-soluble polyamides containing acidic groups are also suitable for this purpose. Derivatives of starch other than those stated above, for example aldehyde starches, may further be used. Cellulose ethers, such as carboxymethylcellulose (Na salt), methylcellulose, hydroxyalkylcellulose and mixed ethers, such as methylhydroxyethylcellulose, methylhydroxypropylcellulose, methylcarboxymethylcellulose and mixtures thereof, are preferably used, for example in quantities of 0.1 to 5 wt.% relative to the compositions.

The washing compositions may contain optical brighteners, among these in particular derivatives of diaminostilbene disulfonic acid or the alkali metal salts thereof. Suitable compounds are, for example, salts of 4,4'-bis(2-anilino-4-morpholino-1,3,5-triazinyl-6-amino)stilbene 2,2'-disulfonic acid or compounds of similar structure which, instead of the morpholino group, bear a diethanolamino group, a methylamino group, an anilino group or a 2-methoxyethylamino group. Brighteners of the substituted diphenylstyryl type may furthermore be present, for example the alkali metal salts of 4,4'-bis(2-sulfostyryl)diphenyl, 4,4'-bis(4-chloro-3-sulfostyryl)diphenyl, or 4-(4-chlorostyryl)-4'-(2-sulfostyryl)diphenyl. Mixtures of the above-stated optical brighteners may also be used.

Especially for use in machine washing or cleaning methods, it may be advantageous to add conventional foam inhibitors to the compositions. Suitable foam inhibitors are, for example, soaps of natural or synthetic origin, which comprise an elevated proportion of $C_{18}$-$C_{24}$ fatty acids. Suitable non-surfactant foam inhibitors are, for example, organopolysiloxanes and mixtures thereof with microfine, optionally silanized silica as well as paraffins, waxes, microcrystalline waxes and mixtures thereof with silanized silica or bis-fatty acid alkylenediamides. Mixtures of different foam inhibitors are also advantageously used, for example mixtures of silicones, paraffins or waxes. The foam inhibitors, in particular foam inhibitors containing silicone and/or paraffin, are preferably bound to a granular carrier substance which is soluble or dispersible in water. Mixtures of paraffins and bistearylethylenediamide are particularly preferred here.

Active ingredients for avoiding tarnishing of silver objects or "silver corrosion inhibitors" may moreover be used in compositions according to the invention. Preferred silver anticorrosion compositions are organic disulfides, dihydric phenols, trihydric phenols, optionally alkyl- or aminoalkyl-substituted triazoles such as benzotriazole and cobalt, manganese, titanium, zirconium, hafnium, vanadium or cerium salts and/or complexes, in which the stated metals are present in one of the oxidation states II, III, IV, V or VI.

The protease variant may assume the form of powder or granules, which may also optionally be coated and/or colored and may contain conventional carrier materials and/or granulation auxiliaries. In the case of use in granule form, the granules may if desired also contain further active substances.

The production of solid compositions according to the invention is unproblematic and may proceed in a manner known in principle, for example by spray drying or granulation, with any peroxy compound and bleach-boosting active ingredient optionally being added subsequently. Compositions according to the invention with an elevated bulk density, in particular in the range from 650 g/l to 950 g/l, may preferably be produced by a method comprising an extrusion step. Washing or cleaning compositions or disinfectants according to the invention in the form of aqueous solutions or solutions containing other conventional solvents are particularly advantageously produced by simply mixing the ingredients, which may be introduced into an automatic mixer without solvent or as a solution. In a preferred embodiment of compositions in particular for automatic dishwashing, the compositions are in tablet form.

**Methods and uses**

The present invention is also directed to the use of a detergent composition as described herein in a cleaning process such as laundry and/or hard surface cleaning. Especially, the present invention is directed to the use of a detergent compositions according to the invention in laundry of textile and fabrics, such as Industrial and Institutional cleaning, house hold laundry washing and industrial laundry washing. Further, the invention is also directed to the use of a detergent compositions according to the invention in hard surface cleaning such as automated Dish Washing (ADW), car wash and cleaning of Industrial surfaces.

One aspect of the disclosure relates to the use of a detergent composition comprising a protease variant, comprising deletion of one amino acids corresponding to positions 53, of the mature polypeptide consisting of SEQ ID NO: 2, wherein the variant has at least 95% identity to SEQ ID NO: 2 in a cleaning process such as laundry and/or hard surface cleaning. Another aspect relates to the use of a detergent composition comprising a variant comprising a deletion of one amino acid corresponding to position 53 of the mature polypeptide consisting of SEQ ID NO: 2 and further comprising one or more substitutions at positions corresponding to positions 54, 55, 56 or 57 of the mature polypeptide of SEQ ID NO: 2, wherein the variant has a sequence identity to SEQ ID NO: 2 of at least 965% and less than 100% and the variant has protease activity.

One aspect of the disclosure relates to the use of a protease variant, comprising deletion of one amino acid corresponding to positions 53 of the mature polypeptide consisting of SEQ ID NO: 2, wherein the variant has at least 95% identity to SEQ ID NO: 2 -in a cleaning process such as laundry and/or hard surface cleaning and wherein the variant has increased wash performance relative to the parent or relative to a protease parent having the identical amino acid sequence of said variant but not having the substitutions at one or more of said positions when tested in the AMSA, as described under "Material and Methods".

The cleaning process or the textile care process may for example be a laundry process, a dishwashing process or cleaning of hard surfaces such as tiles, floors, table-tops, drains, sinks washbasins and surgical instruments. Laundry processes can for example be household laundering, but it may also be industrial laundering.

Furthermore, the disclosure relates to a process for laundering of fabrics and/or garments where the process comprises treating fabrics with a washing solution containing a detergent composition according to the invention. The cleaning process or a textile care process can for example be carried out in a machine-washing process or in a manual washing process. The washing solution can for example be an aqueous washing solution containing a detergent composition.The fabrics and/or garments subjected to a washing, cleaning or textile care process may be conventional washable laundry, for example household laundry. Preferably, the major part of the laundry is garments and fabrics, including knits, woven, denims, non-woven, felts, yarns, and towelling. The fabrics may be cellulose based such as natural cellulosics, including cotton, flax, linen, jute, ramie, sisal or coir or manmade cellulosics (e.g., originating from wood pulp) including viscose/rayon, ramie, cellulose acetate fibers (tricell), lyocell or blends thereof. The fabrics may also be non-cellulose based such as natural polyamides including wool, camel, cashmere, mohair, rabbit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g., polyamide fibers, acrylic fibers, polyester fibers, polyvinyl alcohol fibers, polyvinyl chloride fibers, polyurethane fibers, polyurea fibers, aramid fibers), and cellulose-containing fibers (e.g., rayon/viscose, ramie, flax, linen, jute, cellulose acetate fibers, lyocell).

The last few years there has been an increasing interest in replacing components in detergents, which is derived from petrochemicals with renewable biological components such as enzymes and polypeptides without compromising the wash performance.

The disclosure further concerns the use of protease variants and/or detergent compositions of the invention in a proteinaceous stain removing processes. The proteinaceous stains may be stains such as food stains, e.g., baby food, cocoa, egg and milk or body soiling's as blood and sebum or other soiling's as ink or grass, or a combination hereof.

Typical detergent compositions include various components in addition to the enzymes, these components have different effects, some components like the surfactants lower the surface tension in the detergent, which allows the stain being cleaned to be lifted and dispersed and then washed away, other components like bleach systems remove discolor often by oxidation and many bleaches also have strong bactericidal properties, and are used for disinfecting and sterilizing. Yet other components like builder and chelator softens, e.g., the wash water by removing the metal ions form the liquid.

In a particular embodiment, the invention concerns the use of a detergent composition according to the invention, i.e. comprising a protease variant as described above, in laundry or dish wash, wherein said detergent composition further comprises at least one or more of the following: a surfactant, a builder, a chelator or chelating agent, bleach system and/or bleach component, in particular a surfactant, a builder, a chelator or chelating agent, bleach system and/or bleach component as described above. In another embodiment of said use, the amount of a surfactant, a builder, a chelator or chelating agent, bleach system and/or bleach component are reduced compared to amount of surfactant, builder, chelator or chelating agent, bleach system and/or bleach component used without the added protease variant of the invention. Preferably at least one component which is a surfactant, a builder, a chelator or chelating agent, bleach system and/or bleach component is present in an amount that is 1% less, such as 2% less, such as 3% less, such as 4% less, such as 5% less, such as 6% less, such as 7% less, such as 8% less, such as 9% less, such as 10% less, such as 15% less, such as 20% less, such as 25% less, such as 30% less, such as 35% less, such as 40% less, such as 45% less, such as 50% less than the amount of the component in the system without the addition of protease variant used in the invention, such as a conventional amount of such component. In one aspect, a protease variant used in the invention is used in detergent compositions wherein said composition is free of at least one component which is a surfactant, a builder, a chelator or chelating agent, bleach system or bleach component and/or polymer.

## Washing Method

The detergent compositions of the present invention are ideally suited for use in laundry applications. Accordingly, the present disclosure includes a method for laundering a fabric. The method comprises the steps of contacting a fabric to be laundered with a cleaning laundry solution comprising the detergent composition according to the invention. The fabric may comprise any fabric capable of being laundered in normal consumer use conditions. The solution preferably

has a pH from about 5.5 to about 11.5. The compositions may be employed at concentrations from about 100 ppm, preferably 500 ppm to about 15,000 ppm in solution. The water temperatures typically range from about 5°C to about 95°C, including about 10°C, about 15°C, about 20°C, about 25°C, about 30°C, about 35°C, about 40°C, about 45°C, about 50°C, about 55°C, about 60°C, about 65°C, about 70°C, about 75°C, about 80°C, about 85°C and about 90°C. The water to fabric ratio is typically from about 1:1 to about 30:1.

In particular embodiments, the washing method is conducted at a pH from about 5.0 to about 11.5, or from about 6 to about 10.5, about 5 to about 11, about 5 to about 10, about 5 to about 9, about 5 to about 8, about 5 to about 7, about 5.5 to about 11, about 5.5 to about 10, about 5.5 to about 9, about 5.5 to about 8, about 5.5. to about 7, about 6 to about 11, about 6 to about 10, about 6 to about 9, about 6 to about 8, about 6 to about 7, about 6.5 to about 11, about 6.5 to about 10, about 6.5 to about 9, about 6.5 to about 8, about 6.5 to about 7, about 7 to about 11, about 7 to about 10, about 7 to about 9, or about 7 to about 8, about 8 to about 11, about 8 to about 10, about 8 to about 9, about 9 to about 11, about 9 to about 10, about 10 to about 11, preferably about 5.5 to about 11.5.

In particular embodiments, the washing method is conducted at a degree of hardness of from about 0°dH to about 30°dH, such as about 1°dH, about 2°dH, about 3°dH, about 4°dH, about 5°dH, about 6°dH, about 7°dH, about 8°dH, about 9°dH, about 10°dH, about 11°dH, about 12°dH, about 13°dH, about 14°dH, about 15°dH, about 16°dH, about 17°dH, about 18°dH, about 19°dH, about 20°dH, about 21°dH, about 22°dH, about 23°dH, about 24°dH, about 25°dH, about 26°dH, about 27°dH, about 28°dH, about 29°dH, about 30°dH. Under typical European wash conditions, the degree of hardness is about 16°dH, under typical US wash conditions about 6°dH, and under typical Asian wash conditions, about 3°dH.

The present disclosure relates to a method of cleaning a fabric, a dishware or hard surface with a detergent composition comprising a protease variant used in the invention.

A preferred embodiment concerns a method of cleaning, said method comprising the steps of: contacting an object with a cleaning composition comprising a protease variant used in the invention under conditions suitable for cleaning said object. In a preferred embodiment the cleaning composition is a detergent composition and the process is a laundry or a dish wash process.

Still another embodiment relates to a method for removing stains from fabric which comprises contacting said a fabric with a composition comprising a protease used in the invention under conditions suitable for cleaning said object.

In a preferred embodiment the compositions for use in the methods above further comprise at least one additional enzyme as described above, such as an enzyme selected from the group of hydrolases such as proteases, lipases, cutinases, carbohydrases such as amylases, cellulases, hemicellulases, xylanases, and pectinase or a combination hereof. In yet another preferred embodiment the compositions for use in the methods above comprise a reduced amount of at least one or more of the following components: a surfactant, a builder, a chelator or chelating agent, bleach system or bleach component or a polymer.

Also contemplated are compositions and methods of treating fabrics (e.g., to desize a textile) using one or more of the protease of the invention. The protease can be used in any fabric-treating method which is well known in the art (see, e.g., U.S. Patent No. 6,077,316). For example, in one aspect, the feel and appearance of a fabric is improved by a method comprising contacting the fabric with a protease variant of the invention in a solution. In one aspect, the fabric is treated with the solution under pressure.

In one embodiment, the protease variant is applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The protease variant can be applied to remove these sizing protein or protein derivatives. After the textiles have been woven, a fabric can proceed to a desizing stage. This can be followed by one or more additional fabric processing steps. Desizing is the act of removing size from textiles. After weaving, the size coating should be removed before further processing the fabric in order to ensure a homogeneous and wash-proof result. Also provided is a method of desizing comprising enzymatic hydrolysis of the size by the action of an enzyme.

All issues, subject matter and embodiments which are disclosed for detergent compositions in this application are also applicable for methods and uses described herein. Therefore, it is explicitly referred to said disclosure for the methods and uses described herein as well.

Examples for illustrative purposes only

Materials and Methods

General molecular biology methods:

Unless otherwise mentioned the DNA manipulations and transformations were performed using standard methods of molecular biology (Sambrook et al. (1989); Ausubel et al. (1995); Harwood and Cutting (1990).

Protease assays:

1) Suc-AAPF-pNA assay:

| | |
|---|---|
| pNA substrate | : Suc-AAPF-pNA (Bachem L-1400). |
| Temperature | : Room temperature (25°C) |
| Assay buffers | : 100mM succinic acid, 100mM HEPES, 100mM CHES, 100mM CABS, 1mM $CaCl_2$, 150mM KCl, 0.01% Triton X-100 adjusted to pH-values 2.0, 3.0, 4.0, 5.0, 6.0, 7.0, 8.0, 9.0, 10.0, and 11.0 with HCl or NaOH. |

20μl protease (diluted in 0.01% Triton X-100) was mixed with 100μl assay buffer. The assay was started by adding 100μl pNA substrate (50mg dissolved in 1.0ml DMSO and further diluted 45x with 0.01% Triton X-100). The increase in $OD_{405}$ was monitored as a measure of the protease activity.

2) Protazyme AK assay:

| | |
|---|---|
| Substrate | : Protazyme AK tablet (cross-linked and dyed casein; from Megazyme) |
| Temperature | : 37°C (or set to other assay temperature). |
| Assay buffer | : 100mM succinic acid, 100mM HEPES, 100mM CHES, 100mM CABS, 1mM $CaCl_2$, 150mM KCl, 0.01% Triton X-100, pH 6.5 or pH 7.0. |

A Protazyme AK tablet was suspended in 2.0ml 0.01% Triton X-100 by gentle stirring. 500μl of this suspension and 500μl assay buffer were dispensed in a microcentrifuge tube and placed on ice. 20μl protease solution (diluted in 0.01% Triton X-100) was added to the ice-cold mixture. The assay was initiated by transferring the tube to a thermomixer at 37°C and shaking at its highest rate (1400 rpm.). After 15 minutes the tube was put back into the ice bathTo remove unreacted substrate, the mixture was centrifuged in an ice cold centrifuge for a few minutes and 200μl supernatant was transferred to a microtiter plate. The absorbance of the supernatant at 650 nm was measured. A sample with 20μl of 0.01% Triton X-100 instead of protease solution was assayed in parallel, and its value was subtracted from the protease sample measurement.

Automatic Mechanical Stress Assay (AMSA) for laundry

In order to assess the wash performance in laundry washing experiments were performed, using the Automatic Mechanical Stress Assay (AMSA). With the AMSA, the wash performance of a large quantity of small volume enzyme-detergent solutions can be examined. The AMSA plate has a number of slots for test solutions and a lid firmly squeezing the laundry sample, the textile to be washed against all the slot openings. During the washing time, the plate, test solutions, textile and lid were vigorously shaken to bring the test solution in contact with the textile and apply mechanical stress in a regular, periodic oscillating manner. For further description see WO02/42740 especially the paragraph "Special method embodiments" at page 23-24.

The laundry wash experiments were conducted under the following experimental conditions:

| | |
|---|---|
| **AMSA Method** | ADT-PR-002; AMSA: Automatic Mechanical Stress Assay<br>Employing 384 well plates with 140μl detergent solution and 20μl enzyme in each well.<br>Biomek FX(P) Laboratory Automation Workstation was used for enzyme dilution and dispensing buffer, enzymes and detergent into the AMSA plate; ADT-TE-073. |
| **Detergent** | Detergent 5, DC01650<br>PDET2, DC01652 |

(continued)

| | |
|---|---|
| **Detergent conc.** | Detergent 5: 5 g/l (see details below)<br>PDET2: 2.5 g/L (see details below) |
| **Temperature** | 30°C |
| **Dosages in AMSA-plate** | 140μl detergent per slot; 20μl enzyme per slot |
| **Water hardness** | 15°dH $Ca^{2+}$/$Mg^{2+}$/$NaHCO_3$ 4:1:7.5<br>3.00 ml 0.713mol/L $CaCl_2$ x $2H_2O$<br>1.50 ml 0.357mol/L $MgCl_2$<br>7.50 ml 0.535mol/L $NaHCO_3$<br>875 ml Milli Q water added |
| **Enzyme dosage** | 0 - 10 - 30 nM |
| **Wash time** | 20 min |
| **Stain/ test swatch** | PC-05, Sam-2009-00178. Blood/milk/ink on cotton/polyester<br>PC-03, Sam-2010-00119. Chocolate-milk/soot on cotton/polyester<br>Obtained from Center For Test materials BV, P.O. Box 120, 3133 KT Vlaardingen, the Netherlands |
| **Chemicals** | Succinic acid buffer:<br>10mM Succinic acid + 2mM $CaCl_2$ + 0.02% Brij-35 adjusted to pH 6.5 |
| **AMSA wash Evaluation** | The wash performance was evaluated by scanning the test swatch and transferring the image into intensity values by using the NZ Color Vector Analyzer program. The data were evaluated in the program Excel from Microsoft, calculating relative performances and drawing a chart. |

The following model detergents were used:

| | |
|---|---|
| **Powder model detergent, PDET2** | 20.05 g Na-citrate dehydrate PDC-00097-5<br>15.01 g Na-LAS DC-00010-14<br>20.01 g SLS DC-01181-1<br>These were mixed with a spoon<br>3.98 g Neodol 25-7 (from HMN) was added slowly under stirring<br>3.02 g Na-sulfate PDC-00085-06<br>was added to de-agglomerate the somewhat sticky powder |
| **Liquid model detergent, Detergent 5** | Sodium alkylethoxy sulphate (C-9-15, 2EO) 6.0%<br>Sodium dodecyl benzene sulphonate 3.0%<br>Sodium toluene sulphonate 3.0%<br>Olic acid 2.0%<br>Primary alcohol ethoxylate (C12-15, 7EO) 3.0%<br>Primary alcohol ethoxylate (C12-15, 3EO) 2.5%<br>Ethanol 0.5%<br>Monopropylene glycol 2.0%<br>Tri-sodium citrate 2H2O 4.0%<br>Triethanolamine 0.4%<br>De-ionized water added to 100%<br>pH adjusted to 8.5 with NaOH |

Water hardness was adjusted to 15°dH by addition of $CaCl_2$, $MgCl_2$, and $NaHCO_3$ ($Ca^{2+}$:$Mg^{2+}$ = 4:1:7.5) to the test system. After washing the textiles were flushed in tap water and dried.

The wash performance is measured as the brightness of the colour of the textile washed. Brightness can also be expressed as the intensity of the light reflected from the sample when illuminated with white light. When the sample

is stained the intensity of the reflected light is lower, than that of a clean sample. Therefore the intensity of the reflected light can be used to measure wash performance.

Color measurements were made with a professional flatbed scanner (Kodak iQsmart, Kodak, Midtager 29, DK-2605 Brøndby, Denmark), which is used to capture an image of the washed textile.

To extract a value for the light intensity from the scanned images, 24-bit pixel values from the image were converted into values for red, green and blue (RGB). The intensity value (Int) was calculated by adding the RGB values together as vectors and then taking the length of the resulting vector:

$$Int = \sqrt{r^2 + g^2 + b^2}$$

**Example 1: Preparation and testing of protease variants**

Preparation and Expression of variants

Mutation and introduction of an expression cassette into *Bacillus subtilis.*
All DNA manipulations were done by PCR (e.g. Sambrook et al.; Molecular Cloning; Cold Spring Harbor Laboratory Press) and can be repeated by everybody skilled in the art.
Recombinant B. *subtilis* constructs encoding subtilase variants were used to inoculate shakeflasks containing a rich media (e.g. PS-1: 100 g/L Sucrose (Danisco cat.no. 109-0429), 40 g/L crust soy (soy bean flour), 10g/L $Na_2HPO_4.12H_2O$ (Merck cat.no. 6579), 0.1ml/L Pluronic PE 6100 (BASF 102-3098)). Cultivation typically takes 4 days at 30°C shaking with 220rpm.

Fermentation of variants

Fermentation may be performed by methods well known in the art or as follows. A B. *subtilis* strain harboring the relevant expression plasmid was streaked on a LB-agar plate with a relevant antibiotic (6μg/ml chloramphenicol), and grown overnight at 37°C.

The colonies were transferred to 100 ml PS-1 media supplemented with the relevant antibiotic in a 500 ml shaking flask.

Cells and other undissolved material were removed from the fermentation broth by centrifugation at 4500 rpm for 20-25 minutes. Afterwards the supernatant was filtered to obtain a clear solution.

**Example 2:**

The wash performance of the protease variants and their corresponding protease parent from fermentation supernatants were tested in fermentation supernatants and model detergents at a temperature of 30°C using the AMSA-test method as described under "Material and Methods". PDET2 is a powder model detergent, and Detergent 5 is a liquid model detergent as described under "Material and Methods".

Results:

The relative wash performance of the protease variants and their corresponding protease parent (SEQ ID NO: 2) for two stains PC-03 (Chocolate milk and soot on cotton/polyester) and PC-05 (Blood, milk and ink on cotton/polyester) are shown in Table 2.1 below.

Percent protease wash performance relative to BPN' (SEQ ID NO: 2).

| Variants | PDET2 | | Detergent 5 | |
|---|---|---|---|---|
| | PC-03 | PC-05 | PC-03 | PC-05 |
| BPN' (SEQ ID NO: 2) | 100 | 100 | 100 | 100 |
| S53* | 122 | 110 | - | - |
| E54* | 120 | 109 | - | - |
| T55* | 133 | 122 | 134 | 127 |
| N56* | 140 | 125 | 138 | 137 |

(continued)

| Variants | PDET2 | | Detergent 5 | |
|---|---|---|---|---|
| | PC-03 | PC-05 | PC-03 | PC-05 |
| P57* | 130 | 116 | 124 | 116 |
| S53* + Y217L | 136 | 125 | - | - |
| E54* + Y217L | 119 | 110 | 111 | 106 |
| T55* + Y217L | 143 | 128 | 142 | 140 |
| N56* + Y217L | 138 | 124 | 142 | 140 |
| P57* + Y217L | 139 | 123 | 130 | 125 |
| S53G + T55S + N56* + P57A + Y217L | 137 | 124 | 138 | 138 |
| P14T + T55S + N56* + P57A + Y217L | 138 | 129 | 131 | 137 |
| P14T + S53G + N56* + P57A + Y217L | 138 | 125 | 128 | 135 |
| P14T + S53G + T55S + N56* + Y217L | 136 | 128 | 127 | 137 |
| P14T + S53G + T55S + N56* + P57A | 141 | 134 | 147 | 142 |
| P14T + S53G + T55S + P57A + Y217L | 144 | 129 | 149 | 137 |
| P14T + S53G + T55S + N56* + P57A+ S101L + Y217L | 128 | 125 | 114 | 142 |
| V4I + S53G + T55S + N56* + P57A + Y217L | 142 | 129 | 139 | 147 |
| P14T + S53G + T55S + N56* + P57A + Y217L | 142 | 131 | 142 | 146 |
| T55S + N56* + P57A + Y217L | 150 | 134 | 144 | 149 |
| S53G + T55S + N56* + P57A + I79T + Y217L | 143 | 131 | 141 | 145 |
| S53G + T55S + N56* + P57A + P86H + A92S + Y217L | 134 | 121 | 134 | 143 |
| S53G + T55S + N56* + P57A + A88V + Y217L | 133 | 121 | 140 | 137 |
| S53G + T55S + N56* + P57A + A98T + Y217L | 142 | 127 | 136 | 137 |
| S53G + T55S + N56* + P57A + Y217L | 141 | 120 | 140 | 157 |
| S53G + T55P + N56* + S63G + G146S + Y217L | 138 | 117 | 132 | 130 |
| Y217L | 119 | 110 | 109 | 109 |

Percent protease wash performance relative to BPN' Y217L is shown in Table 2.2.

| Variants | PDET2 | | Detergent 5 | |
|---|---|---|---|---|
| | PC-03 | PC-05 | PC-03 | PC-05 |
| BPN' (SEQ ID NO: 2) | 84 | 91 | 92 | 91 |
| Y217L | 100 | 100 | 100 | 100 |
| S53* | 103 | 100 | - | - |
| E54* | 101 | 99 | - | - |
| T55* | 112 | 111 | 123 | 116 |
| N56* | 118 | 114 | 126 | 125 |
| P57* | 109 | 106 | 114 | 106 |
| S53* + Y217L | 115 | 114 | - | - |
| E54* + Y217L | 100 | 100 | 102 | 97 |

(continued)

| Variants | PDET2 | | Detergent 5 | |
|---|---|---|---|---|
| T55* + Y217L | 121 | 117 | 130 | 127 |
| N56* + Y217L | 117 | 113 | 131 | 127 |
| P57* + Y217L | 117 | 112 | 119 | 114 |
| S53G + T55S + N56* + P57A + Y217L | 115 | 113 | 127 | 126 |
| P14T + T55S + N56* + P57A + Y217L | 116 | 117 | 120 | 125 |
| P14T + S53G + N56* + P57A + Y217L | 117 | 114 | 118 | 124 |
| P14T + S53G + T55S + N56* + Y217L | 114 | 117 | 117 | 125 |
| P14T + S53G + T55S + N56* + P57A | 118 | 122 | 135 | 129 |
| P14T + S53G + T55S + P57A + Y217L | 121 | 117 | 136 | 125 |
| P14T + S53G + T55S + N56* + P57A + S101L + Y217L | 108 | 114 | 105 | 130 |
| V4I + S53G + T55S + N56* + P57A + Y217L | 120 | 117 | 128 | 134 |
| P14T + S53G + T55S + N56* + P57A + Y217L | 119 | 119 | 130 | 134 |
| T55S + N56* + P57A + Y217L | 126 | 122 | 132 | 136 |
| S53G + T55S + N56* + P57A + I79T + Y217L | 120 | 119 | 130 | 133 |
| S53G + T55S + N56* + P57A + P86H + A92S + Y217L | 113 | 111 | 123 | 131 |
| S53G + T55S + N56* + P57A + A88V + Y217L | 112 | 110 | 128 | 125 |
| S53G + T55S + N56* + P57A + A98T + Y217L | 120 | 116 | 125 | 125 |
| S53G + T55S + N56* + P57A + Y217L | 119 | 105 | 129 | 143 |
| S53G + T55P + N56* + S63G + G146S + Y217L | 116 | 107 | 121 | 119 |

As the two tables above show the wash performance of all investigated variants are increased relative to the BPN' (SEQ ID NO: 2). A deletion of position 55, 56 or 57 significantly and substantially improved wash performance. Improved wash performance is observed when a deletion of position 53 or 54 is present. In addition substitutions in the loop region result in a significantly improved wash performance. Substitutions in neighboring positions to the deletion in the loop result in slightly further improved wash performance. The tested variants that contain additional mutations outside the loop corresponding to positions 53, 54, 55, 56 or 57, such as Y217L, of the mature polypeptide of SEQ ID NO: 2 show at least as good wash performance as their parent without this additional mutation.

**Example 3:**

The wash performance of detergents according to the invention was determined by using the following standardized stains:

A: chocolate milk and soot on cotton: product no. C-03 obtainable from CFT (Center for Testmaterials) B.V., Vlaardingen, Netherlands,
B: blood, milk, ink on cotton: product no. C-05 obtainable from CFT (Center for Testmaterials) B.V., Vlaardingen, Netherlands,
C: chocolate milk and soot on polyester/cotton: product no. PC-03 obtainable from CFT (Center for Testmaterials) B.V., Vlaardingen, Netherlands,
D: blood, milk, ink on polyester/cotton: product no. PC-05 obtainable from CFT (Center for Testmaterials) B.V., Vlaardingen, Netherlands,
E: grass on cotton: product no. 164 obtainable from Eidgenössische Material- und Prüfanstalt (EMPA) Testmaterialien AG [Federal materials and testing agency, Testmaterials], St. Gallen, Switzerland.

A liquid washing agent with the following composition was used as base formulation (all values in weight

percent): 0.3 to 0.5% xanthan gum, 0.2 to 0.4% antifoaming agent, 6 to 7% glycerol, 0.3 to 0.5% ethanol, 4 to 7% FAEOS (fatty alcohol ether sulfate), 24 to 28% nonionic surfactants, 1% boric acid, 1 to 2% sodium citrate (dihydrate), 2 to 4% soda, 14 to 16% coconut fatty acid, 0.5% HEDP (1-hydroxyethane-(1,1-diphosphonic acid)), 0 to 0.4% PVP (polyvinylpyrrolidone), 0 to 0.05% optical brighteners, 0 to 0.001 % dye, remainder deionized water.

Based on this base formulation, various detergents according to the invention were prepared by adding respective proteases as indicated in tables 3.1 and 3.2. The BPN' variant BPN' Y217L was used as reference, the reference protease already showing a good wash performance, especially in liquid detergents. The proteases were added in the same amounts based on total protein content (5 mg/l wash liquor).

The dosing ratio of the liquid washing agent was 4.7 grams per liter of washing liquor and the washing procedure was performed for 60 minutes at a temperature of 20°C and 40°C, the water having a water hardness between 15.5 and 16.5° (German degrees of hardness).

The whiteness, i.e. the brightening of the stains, was determined photometrically as an indication of wash performance. A Minolta CM508d spectrometer device was used, which was calibrated beforehand using a white standard provided with the unit.

The results obtained are the difference values between the remission units obtained with the detergent according to the disclosure and the remission units obtained with the detergent containing the reference protease. A positive values therefore indicates an improved wash performance of the detergent of the disclosure. It is evident from tables 3.1 (results at 40°C) and 3.2 (results at 20°C) that detergents according to the disclosure show improved wash performance.

Table 3.1:

| Protease variant / stain | A | B | C | D | E |
|---|---|---|---|---|---|
| V4I + S53G + T55S + N56* + P57A + Y217L | 2.0 | 2.4 | 1.0 | 4.3 | 0.9 |
| P14T + S53G + T55S + N56* + P57A + Y217L | 1.8 | 3.2 | 1.3 | 4.7 | 0.6 |
| T55S + N56* + P57A + Y217L | 1.3 | 3.7 | 1.5 | 3.6 | 0.1 |
| S53G + T55S + N56* + P57A + I79T + Y217L | 1.7 | 2.9 | 2.0 | 4.8 | 1.1 |
| S53G + T55S + N56* + P57A + V84I + Y217L | 1.0 | 3.4 | 1.2 | 3.9 | 0.6 |
| S53G + T55S + N56* + P57A + P86H + A92S + Y217L | 0.4 | 3.7 | 0.7 | 3.2 | 0.5 |
| S53G + T55S + N56* + P57A + A88V + Y217L | 1.7 | 2.7 | 1.1 | 4.2 | 0.7 |
| S53G + T55S + N56* + P57A + A98T + Y217L | 2.3 | 3.0 | 1.4 | 4.5 | 1.4 |
| S53G + T55S + N56* + P57A + N118R + Y217L | 1.1 | 0.7 | 2.0 | 0.5 | 0.2 |
| S53G + T55S + N56* + P57A + G97D + Y217L | 2.5 | 1.8 | 2.7 | 2.3 | 1.2 |
| S53G + T55S + N56* + P57A + S101N + Y217L | 1.8 | 1.6 | 1.1 | 1.9 | 0.4 |
| S53G + T55S + N56* + P57A + G110A + Y217L | 3.2 | 0.8 | 3.5 | 2.3 | 1.5 |

Table 3.2:

| Protease variant / stain | A | B | C | D | E |
|---|---|---|---|---|---|
| V4I + S53G + T55S + N56* + P57A + Y217L | 1.1 | 0.3 | 3.1 | 3.9 | 0.5 |
| P14T + S53G + T55S + N56* + P57A + Y217L | 2.0 | 1.0 | 3.3 | 4.5 | 0.9 |
| T55S + N56* + P57A + Y217L | 1.6 | 0.0 | 3.5 | 3.6 | 0.6 |
| S53G + T55S + N56* + P57A + I79T + Y217L | 1.0 | 0.2 | 3.4 | 4.0 | 0.1 |
| S53G + T55S + N56* + P57A + V84I + Y217L | 0.8 | 0.4 | 2.8 | 3.8 | 1.1 |
| S53G + T55S + N56* + P57A + P86H + A92S + Y217L | 1.3 | 0.1 | 1.5 | 3.6 | 1.2 |
| S53G + T55S + N56* + P57A + A88V + Y217L | 0.8 | 0.5 | 3.5 | 4.0 | 0.9 |
| S53G + T55S + N56* + P57A + A98T + Y217L | 1.1 | 0.1 | 3.9 | 4.7 | 1.5 |
| S53G + T55S + N56* + P57A + N118R + Y217L | 0.6 | 0.8 | 0.9 | 1.3 | 0.5 |

(continued)

| Protease variant / stain | A | B | C | D | E |
|---|---|---|---|---|---|
| H17Y + S53G + T55S + N56* + P57A + Y217L | 2.3 | 0.8 | 1.4 | 2.6 | 1.0 |
| S53G + T55S + N56* + P57A + G97D + Y217L | 0.7 | 0.9 | 3.6 | 2.3 | 0.1 |
| S53G + T55S + N56* + P57A + S101N + Y217L | 0.1 | 0.9 | 3.2 | 2.2 | 0.5 |
| S53G + T55S + N56* + P57A + G110A + Y217L | 1.9 | 0.6 | 4.4 | 2.0 | 0.1 |

The invention described and claimed herein is not to be limited in scope by the specific aspects herein disclosed, since these aspects are intended as illustrations of several aspects of the invention.

## Claims

1. A method for producing a detergent composition which comprises the step of adding a protease variant which was obtained by a method comprising introducing into a parent subtilase a deletion at a position corresponding to position 53 of the mature polypeptide consisting of SEQ ID NO: 2 and an additional deletion of one or more amino acids selected from the group consisting of Ser, Glu, Thr, Asn or Pro in the loop corresponding to positions 54, 55, 56 or 57 of the mature polypeptide consisting of SEQ ID NO: 2, wherein the variant has at least 95% identity to SEQ ID NO: 2.

2. The method of claim 1 wherein the variant has at least 95% identity, at least 96%, at least 97%, at least 98%, or at least 99%, but less than 100%, sequence identity to the mature polypeptide consisting of SEQ ID NO: 2.

3. The method of any of claims 1-3, wherein the parent subtilase is selected from the group consisting of:

   a. a polypeptide having at least 95% sequence identity to the mature polypeptide consisting of SEQ ID NO: 2;
   b. a polypeptide encoded by a polynucleotide that hybridizes under medium or high stringency conditions with (i) the mature polypeptide coding sequence consisting of nucleotides 322 to 1146 of SEQ ID NO: 1, (ii) a sequence encoding the mature polypeptide consisting of SEQ ID NO: 2, or (iii) the full-length complement of (i) or (ii);
   c. a polypeptide encoded by a polynucleotide having at least 70% identity to the mature polypeptide coding sequence consisting of nucleotides 322 to 1146 of SEQ ID NO: 1 or a sequence encoding the mature polypeptide consisting of SEQ ID NO: 2; and
   d. a fragment of the mature polypeptide consisting of SEQ ID NO: 2, which has protease activity.

4. A detergent composition comprising a protease variant comprising a deletion at one amino acid corresponding to position 53 of the mature polypeptide consisting of SEQ ID NO: 2 and further comprising one or more deletions at positions corresponding to positions 54, 55, 56 or 57 of the mature polypeptide consisting of SEQ ID NO: 2, wherein

   (a) the variant has at least 95% and less than 100% identity to SEQ ID NO:2 and
   (b) the variant has protease activity.

5. The detergent composition of claim 4, wherein the variant comprises a deletion at a position corresponding to position 53 of SEQ ID NO: 2 and further comprises a substitution at one or more positions corresponding to positions 54, 55, 56 or 57 of the mature polypeptide consisting of SEQ ID NO: 2, and
   wherein the variant further comprises a deletion at a position corresponding to position 54 of SEQ ID NO: 2, and/or wherein the variant further comprises a deletion at a position corresponding to position 55 of SEQ ID NO: 2, and/or wherein the variant further comprises a deletion at a position corresponding to position 56 of SEQ ID NO: 2, and/or wherein the variant further comprises a deletion at a position corresponding to position 57 of SEQ ID NO: 2.

6. The detergent composition of claims 4 or 5,
   wherein the amino acid in the variant at the position corresponding to position 54 of SEQ ID NO: 2 is selected among Gly, Ala, Ser, Thr, Asn or is not present, and/or
   wherein the amino acid in the variant at the position corresponding to position 55 of SEQ ID NO: 2 is selected among Gly, Ala, Ser, Asn or is not present, and/or

wherein the amino acid in the variant at the position corresponding to position 56 of SEQ ID NO: 2 is selected among Gly, Ala, Ser, Thr or is not present, or
wherein the amino acid in the variant at the position corresponding to position 57 of SEQ ID NO: 2 is selected among Gly, Ala, Ser, Thr, Asn or is not present.

**7.** The detergent composition of any of claims 4-6, wherein the variant comprises an alteration at three positions corresponding to any of positions 53, 54, 55, 56, and 57 of SEQ ID NO:2, wherein one of the alterations is a deletion at a position corresponding to position 53 of SEQ ID NO: 2.

**8.** The detergent composition of any of claims 4-7, wherein the variant comprises the deletion S53* and one or more further alterations selected from the group consisting of the deletions E54*, T55*, N56* and P57* and/or the substitutions T55S and P57A, wherein the numbering of the positions is according to SEQ ID NO:2.

**9.** The detergent composition of any of claims 4-8, which has an improved wash performance compared to an identical detergent composition except for the protease being the parent subtilase instead of the variant or to an identical detergent composition except for the protease being the protease with SEQ ID NO: 2 instead of the variant.

**10.** The detergent composition of any of claims 4-9, wherein the variant is selected from the group consisting of:

a. a polypeptide encoded by a polynucleotide that hybridizes under medium, or high stringency conditions with (i) the mature polypeptide coding sequence consisting of nucleotides 322 to 1146 of SEQ ID NO: 1, (ii) a sequence encoding the mature polypeptide consisting of SEQ ID NO: 2, or (iii) the full-length complement of (i) or (ii);
b. a polypeptide encoded by a polynucleotide having at least 70% identity to the mature polypeptide coding sequence consisting of nucleotides 322 to 1146 of SEQ ID NO: 1 or a sequence encoding the mature polypeptide consisting of SEQ ID NO: 2; and
c. a fragment of the mature polypeptide consisting of SEQ ID NO: 2, which has protease activity.

**11.** The use of a detergent composition according to any of claims 4-10 in a cleaning process.

**12.** The use according to claim 11 wherein the cleaning process is laundry.

**13.** The use according to claim 12 wherein the cleaning process is hard surface cleaning such as dish washing.

## Patentansprüche

**1.** Verfahren zum Herstellen einer Reinigungsmittelzusammensetzung, die umfasst: den Schritt des Hinzufügens einer Proteasevariante, die durch ein Verfahren gewonnen worden ist, umfassend das Einführen einer Deletion in eine Ausgangssubtilase an einer Position, die Position 53 des reifen Polypeptids bestehend aus SEQ ID NO: 2 entspricht, und einer zusätzlichen Deletion einer oder mehrerer Aminosäuren, ausgewählt aus der Gruppe bestehend aus Ser, Glu, Thr, Asn oder Pro in der Schleife, die Positionen 54, 55, 56 oder 57 des reifen Polypeptids bestehend aus SEQ ID NO: 2 entsprechen, wobei die Variante eine mindestens 95%ige Identität mit SEQ ID NO: 2 aufweist.

**2.** Verfahren nach Anspruch 1, wobei die Variante eine mindestens 95%ige Identität, mindestens 96%ige, mindestens 97%ige, mindestens 98%ige oder mindestens 99%ige, aber weniger als 100%ige Sequenzidentität mit dem reifen Polypeptid bestehend aus SEQ ID NO: 2 aufweist.

**3.** Verfahren nach einem der Ansprüche 1-3, wobei die Ausgangssubtilase ausgewählt ist aus der Gruppe bestehend aus:

a. einem Polypeptid mit mindestens 95%iger Sequenzidentität mit dem reifen Polypeptid bestehend aus SEQ ID NO: 2;
b. einem Polypeptid, das codiert wird durch ein Polynukleotid, das unter mittleren oder hohen Stringenzbedingungen mit (i) der Codierungssequenz des reifen Polypeptids bestehend aus Nukleotiden 322 bis 1146 von SEQ ID NO: 1, (ii) einer Sequenz, die für das reife Polypeptid bestehend aus SEQ ID NO: 2 codiert, oder (iii) dem Komplement voller Länge aus (i) oder (ii) hybridisiert;
c. einem Polypeptid, das codiert wird durch ein Polynukleotid mit mindestens 70%iger Identität mit der Codierungssequenz des reifen Polypeptids bestehend aus Nukleotiden 322 bis 1146 aus SEQ ID NO: 1, oder einer Sequenz, die für das reife Polypeptid codiert, bestehend aus SEQ ID NO: 2;
d. einem Fragment des reifen Polypeptids bestehend aus SEQ ID NO: 2, das eine Proteaseaktivität aufweist.

**4.** Reinigungsmittelzusammensetzung, umfassend eine Proteasevariante, umfassend eine Deletion an einer Aminosäure, die Position 53 des reifen Polypeptids bestehend aus SEQ ID NO: 2 entspricht, und ferner umfassend eine oder mehrere Deletionen an Positionen, die Positionen 54, 55, 56 oder 57 des reifen Polypeptids bestehend aus SEQ ID NO: 2 entsprechen, wobei

(a) die Variante eine mindestens 95%ige und weniger als 100%ige Identität mit SEQ ID NO: 2 aufweist und
(b) die Variante eine Proteaseaktivität aufweist.

**5.** Reinigungsmittelzusammensetzung nach Anspruch 4, wobei die Variante eine Deletion an einer Position, die Position 53 von SEQ ID NO: 2 entspricht, umfasst und ferner eine Substitution an einer oder mehreren Positionen, die Positionen 54, 55, 56 oder 57 des reifen Polypeptids bestehend aus SEQ ID NO: 2 entsprechen, umfasst, und
wobei die Variante ferner eine Deletion an einer Position, die Position 54 von SEQ ID NO: 2 entspricht, umfasst und/oder
wobei die Variante ferner eine Deletion an einer Position, die Position 55 von SEQ ID NO: 2 entspricht, umfasst und/oder
wobei die Variante ferner eine Deletion an einer Position, die Position 56 von SEQ ID NO: 2 entspricht, umfasst und/oder
wobei die Variante ferner eine Deletion an einer Position, die Position 57 von SEQ ID NO: 2 entspricht, umfasst.

**6.** Reinigungsmittelzusammensetzung nach Anspruch 4 oder 5,
wobei die Aminosäure in der Variante an der Position, die Position 54 von SEQ ID NO: 2 entspricht, ausgewählt ist aus Gly, Ala, Ser, Thr, Asn oder nicht vorhanden ist und/oder
wobei die Aminosäure in der Variante an der Position, die Position 55 von SEQ ID NO: 2 entspricht, ausgewählt ist aus Gly, Ala, Ser, Asn oder nicht vorhanden ist und/oder
wobei die Aminosäure in der Variante an der Position, die Position 56 von SEQ ID NO: 2 entspricht, ausgewählt ist aus Gly, Ala, Ser, Thr oder nicht vorhanden ist und/oder
wobei die Aminosäure in der Variante an der Position, die Position 57 von SEQ ID NO: 2 entspricht, ausgewählt ist aus Gly, Ala, Ser, Thr, Asn oder nicht vorhanden ist.

**7.** Reinigungsmittelzusammensetzung nach einem der Ansprüche 4-6, wobei die Variante eine Änderung an drei Positionen, die beliebigen von Positionen 53, 54, 55, 56 und 57 von SEQ ID NO: 2 entsprechen, umfasst und wobei eine der Änderungen eine Deletion an einer Position, die Position 53 von SEQ ID NO: 2 entspricht, umfasst.

**8.** Reinigungsmittelzusammensetzung nach einem der Ansprüche 4-7, wobei die Variante die Deletion S53* und eine oder mehrere Änderungen, ausgewählt aus der Gruppe bestehend aus den Deletionen E54*, T55*, N56* und P57* und/oder den Substitutionen T55S und P57A, umfasst, wobei die Nummerierung der Positionen gemäß SEQ ID NO: 2 ist.

**9.** Reinigungsmittelzusammensetzung nach einem der Ansprüche 4-8, die verglichen mit einer identischen Reinigungsmittelzusammensetzung, abgesehen davon, dass die Protease die Stammsubtilase anstelle der Variante ist, oder verglichen mit einer identischen Reinigungsmittelzusammensetzung, abgesehen davon, dass die Protease die Protease mit der SEQ ID NO: 2 anstelle der Variante ist, eine verbesserte Waschleistung aufweist.

**10.** Reinigungsmittelzusammensetzung nach einem der Ansprüche 4-9, wobei die Variante ausgewählt ist aus der Gruppe bestehend aus:

a. einem Polypeptid, das codiert wird durch ein Polynukleotid, das unter mittleren oder hohen Stringenzbedingungen mit (i) der Codierungssequenz des reifen Polypeptids bestehend aus Nukleotiden 322 bis 1146 von SEQ ID NO: 1, (ii) einer Sequenz, die für das reife Polypeptid bestehend aus SEQ ID NO: 2 codiert, oder (iii) dem Komplement voller Länge aus (i) oder (ii) hybridisiert;
b. einem Polypeptid, das codiert wird durch ein Polynukleotid mit mindestens 70%iger Identität mit der Codierungssequenz des reifen Polypeptids bestehend aus Nukleotiden 322 bis 1146 aus SEQ ID NO: 1, oder einer Sequenz, die für das reife Polypeptid bestehend aus SEQ ID NO: 2 codiert; und
c. einem Fragment des reifen Polypeptids bestehend aus SEQ ID NO: 2, das eine Proteaseaktivität aufweist.

**11.** Verwendung einer Reinigungsmittelzusammensetzung nach einem der Ansprüche 4-10 in einem Reinigungsvorgang.

**12.** Verwendung nach Anspruch 11, wobei der Reinigungsvorgang Wäschewaschen ist.

**13.** Verwendung nach Anspruch 12, wobei der Reinigungsvorgang Reinigen von harten Oberflächen ist, wie etwa Geschirrspülen.

**Revendications**

**1.** Procédé de production d'une composition détergente qui comprend l'étape d'ajout d'un variant de protéase qui a été obtenu par un procédé comprenant l'introduction dans une subtilase parente d'une délétion à une position correspondant à la position 53 du polypeptide mature constitué de SEQ ID NO: 2 et une délétion supplémentaire d'un ou de plusieurs acides aminés choisis dans le groupe comportant Ser, Glu, Thr, Asn ou Pro dans la boucle correspondant aux positions 54, 55, 56 ou 57 du polypeptide mature consistant en SEQ ID NO: 2, le variant ayant au moins 95 % d'identité avec SEQ ID NO: 2.

**2.** Procédé selon la revendication 1, dans lequel le variant a une identité de séquence d'au moins 95 %, d'au moins 96 %, d'au moins 97 %, d'au moins 98 %, ou d'au moins 99 %, mais de moins de 100 %, avec le polypeptide mature consistant en SEQ ID NO: 2.

**3.** Procédé selon l'une quelconque des revendications 1 à 3, dans lequel la subtilase parente est choisie dans le groupe comprenant :

a. un polypeptide ayant une identité de séquence d'au moins 95 % avec le polypeptide mature consistant en SEQ ID NO: 2 ;
b. un polypeptide codé par un polynucléotide qui s'hybride dans des conditions de stringence moyenne ou élevée avec (i) la séquence codant le polypeptide mature consistant en nucléotides 322 à 1146 de SEQ ID NO: 1, (ii) une séquence codant le polypeptide mature consistant en SEQ ID NO: 2, ou (iii) le complément complet de (i) ou de (ii) ;
c. un polypeptide codé par un polynucléotide ayant une identité d'au moins 70 % avec la séquence codant le polypeptide mature consistant en les nucléotides 322 à 1146 de SEQ ID NO: 1 ou une séquence codant le polypeptide mature de SEQ ID NO: 2 ; et
d. un fragment du polypeptide mature consistant en SEQ ID NO: 2, qui a une activité de protéase.

**4.** Composition détergente comprenant un variant de protéase comprenant une délétion au niveau d'un acide aminé correspondant à la position 53 du polypeptide mature consistant en SEQ ID NO: 2 et comprenant en outre une ou plusieurs délétions à des positions correspondant aux positions 54, 55, 56 ou 57 du polypeptide mature de SEQ ID NO: 2,

(a) le variant ayant une identité d'au moins 95 % et de moins de 100 % avec SEQ ID NO: 2 et
(b) le variant ayant une activité de protéase.

**5.** Composition détergente selon la revendication 4, dans laquelle
le variant comprend une délétion à une position correspondant à la position 53 de SEQ ID NO: 2 et comprend en outre une substitution à une ou plusieurs positions correspondant aux positions 54, 55, 56 ou 57 du polypeptide mature consistant en SEQ ID NO: 2, et
le variant comprend en outre une délétion à une position correspondant à la position 54 de SEQ ID NO: 2 et/ou
le variant comprend en outre une délétion à une position correspondant à la position 55 de SEQ ID NO: 2 et/ou
le variant comprend en outre une délétion à une position correspondant à la position 56 de SEQ ID NO: 2 et/ou
le variant comprend en outre une délétion à une position correspondant à la position 57 de SEQ ID NO: 2.

**6.** Composition détergente selon les revendications 4 ou 5, dans laquelle
l'acide aminé dans le variant à la position correspondant à la position 54 de SEQ ID NO: 2 est sélectionné parmi Gly, Ala, Ser, Thr, Asn ou n'est pas présent, et/ou l'acide aminé dans le variant à la position correspondant à la position 55 de SEQ ID NO: 2 est sélectionné parmi Gly, Ala, Ser, Asn ou n'est pas présent, et/ou l'acide aminé dans le variant à la position correspondant à la position 56 de SEQ ID NO: 2 est sélectionné parmi Gly, Ala, Ser, Thr ou

n’est pas présent, et/ou l’acide aminé dans le variant à la position correspondant à la position 57 de SEQ ID NO: 2 est sélectionné parmi Gly, Ala, Ser, Thr, Asn ou n’est pas présent.

**7.** Composition détergente selon l’une quelconque des revendications 4 à 6, dans laquelle
le variant comprend une altération à trois des positions 53, 54, 55, 56 et 57 de SEQ ID NO: 2, une des altérations étant une délétion à une position correspondant à la position 53 de SEQ ID NO: 2.

**8.** Composition détergente selon l’une quelconque des revendications 4 à 7, dans laquelle
le variant comprend la délétion S53* et une ou plusieurs autres altérations choisie dans le groupe comportant les délétions E54*, T55*, N56* et P57* et/ou les substitutions T55S et P57A, la numérotation correspondant aux positions selon SEQ ID NO: 2.

**9.** Composition détergente selon l’une quelconque des revendications 4 à 8, qui a un pouvoir lavant amélioré en comparaison avec une composition détergente identique, sauf que la protéase est la subtilase parente au lieu du variant, ou avec une composition détergente identique sauf que la protéase est la protéase ayant SEQ ID NO: 2 au lieu du variant.

**10.** Composition détergente selon l’une quelconque des revendications 4 à 9, dans laquelle
le variant est choisi dans le groupe comportant :

a. un polypeptide codé par un polynucléotide qui s’hybride dans des conditions de stringence moyenne ou élevée avec (i) la séquence codant le polypeptide mature consistant en les nucléotides 322 à 1146 de SEQ ID NO: 1, (ii) une séquence codant le polypeptide mature consistant en SEQ ID NO: 2, ou (iii) le complément complet de (i) ou de (ii) ;
b. un polypeptide codé par un polynucléotide ayant une identité d’au moins 70 % avec la séquence codant le polypeptide mature consistant en les nucléotides 322 à 1146 de SEQ ID NO: 1 ou une séquence codant le polypeptide mature consistant en SEQ ID NO: 2; et
c. un fragment du polypeptide mature consistant en SEQ ID NO: 2, qui a une activité de protéase.

**11.** Utilisation d’une composition détergente selon l’une quelconque des revendications 4 à 10 dans un processus de nettoyage.

**12.** Utilisation selon la revendication 11, dans laquelle le processus de nettoyage est une lessive.

**13.** Utilisation selon la revendication 12, dans lequel le processus de nettoyage est le nettoyage d’une surface dure, comme le lavage de la vaisselle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004041979 A
- US 6436690 B
- WO 2009149200 A
- WO 200231133 A
- WO 2011072117 A
- US 20040171154 A
- WO 9517413 A
- WO 9522625 A
- US 5223409 A
- WO 9206204 A
- WO 9219709 A
- WO 9219708 A
- WO 2005105826 A
- WO 2009118375 A
- DE 2412837
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 8906279 A
- WO 9318140 A
- WO 98020115 A
- WO 0144452 A
- WO 0158275 A
- WO 0158276 A
- WO 03006602 A
- WO 04099401 A
- WO 9523221 A
- WO 9221760 A
- EP 1921147 A
- EP 1921148 A
- WO 8906270 A
- WO 9425583 A
- WO 9219729 A
- WO 9820115 A
- WO 9820116 A
- WO 9834946 A
- WO 07044993 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 2007087508 A
- WO 2009109500 A
- GB 1296839 A
- WO 9402597 A
- WO 9418314 A
- WO 9623873 A
- WO 9743424 A
- WO 9324618 A
- WO 9510602 A
- WO 9815257 A
- US 4106991 A
- US 4661452 A
- GB 1483591 A
- EP 238216 A
- US 6077316 A
- WO 0242740 A
- PT 019568
- EP 11194520 A

**Non-patent literature cited in the description**


- *Eur. J. Biochem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **EDGAR.** *Nucleic Acids Research,* 2004, vol. 32, 1792-1797
- **KATOH ; KUMA.** *Nucleic Acids Research,* 2002, vol. 30, 3059-3066
- **KATOH et al.** *Nucleic Acids Research,* 2005, vol. 33, 511-518
- **KATOH ; TOH.** *Bioinformatics,* 2007, vol. 23, 372-374
- **KATOH et al.** *Methods in Molecular Biology,* 2009, vol. 537, 39-64
- **KATOH ; TOH.** *Bioinformatics,* 2010, vol. 26, 1899-1900
- **THOMPSON et al.** *Nucleic Acids Research,* 1994, vol. 22, 4673-4680
- **LINDAHL ; ELOFSSON.** *J. Mol. Biol.,* 2000, vol. 295, 613-615
- **ATSCHUL et al.** *Nucleic Acids Res.,* 1997, vol. 25, 3389-3402
- **JONES.** *J. Mol. Biol.,* 1999, vol. 287, 797-815
- **MCGUFFIN ; JONES.** *Bioinformatics,* 2003, vol. 19, 874-881
- **GOUGH et al.** *J. Mol. Biol.,* 2000, vol. 313, 903-919
- **HOLM ; SANDER.** *Proteins,* 1998, vol. 33, 88-96
- **SHINDYALOV ; BOURNE.** *Protein Engineering,* 1998, vol. 11, 739-747
- **HOLM ; PARK.** *Bioinformatics,* 2000, vol. 16, 566-567
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett,* 1992, vol. 309, 59-64
- **SIEZEN et al.** *Protein Engng,* 1991, vol. 4, 719-737
- **WHITE ; HANDLER ; SMITH.** Principles of Biochemistry. McGraw-Hill Book Company, 1973, 271-272
- **PRIEST.** *Bacteriological Rev.,* 1977, vol. 41, 711-753
- **SIEZEN et al.** *Protein Engng.,* 1991, vol. 4, 719-737
- **SIEZEN et al.** *Protein Science,* 1997, vol. 6, 501-523
- **J.E. NESS et al.** *Nature Biotechnology,* 1999, vol. 17, 893-896
- **NILSSON et al.** *EMBO J.,* 1985, vol. 4, 1075
- **NILSSON et al.** *Methods Enzymol.,* 1991, vol. 198, 3
- **FORD et al.** *Protein Expression and Purification,* 1991, vol. 2, 95-107
- **SAMBROOK et al.** Molecular Cloning, A Laboratory Manual. Cold Spring Harbor, 1989
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol,* 2000, vol. 76, 245-251
- **RASMUSSEN-WILSON et al.** *Appl. Environ. Microbiol,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **SCHERER ; DAVIS.** *Proc. Natl. Acad. Sci. USA,* 1979, vol. 76, 4949-4955
- **BARTON et al.** *Nucleic Acids Res.,* 1990, vol. 18, 7349-4966
- **STORICI et al.** *Nature Biotechnol.,* 2001, vol. 19, 773-776
- **KREN et al.** *Nat. Med.,* 1998, vol. 4, 285-290
- **CALISSANO ; MACINO.** *Fungal Genet. Newslett.,* 1996, vol. 43, 15-16
- **TIAN et al.** *Nature,* 2004, vol. 432, 1050-1054
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156

- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **A. G. GORNALL ; C. S. BARDAWILL ; M.M. DAVID.** *J. Biol. Chem.,* 1948, vol. 177, 751-766
- **DARTOIS et al.** *Biochemica et Biophysica Acta,* 1993, vol. 1131, 253-360
- **SAMBROOK et al.** Molecular Cloning. Cold Spring Harbor Laboratory Press